# EUROPÄISCHE PATENTSCHRIFT

(11) **EP 0 819 255 B1**
(45) Veröffentlichungstag und Bekanntmachung des Hinweises auf die Patenterteilung: **08.10.2003**
(21) Anmeldenummer: 96909143.8
(22) Anmeldetag: 28.03.1996
(51) Int. Cl.: G01N 35/00, B01L 7/00, C12Q 1/68

(54) **SYSTEM ZUR FREISETZUNG UND ISOLIERUNG VON NUKLEINSÄUREN**
SYSTEM FOR RELEASING AND ISOLATING NUCLEIC ACIDS
SYSTEME PERMETTANT DE LIBERER ET D'ISOLER DES ACIDES NUCLEIQUES

(30) Priorität: 01.04.1995 DE 19512368
(43) Veröffentlichungstag der Anmeldung: 21.01.1998
(73) Patentinhaber: Roche Diagnostics GmbH, 68298 Mannheim (DE)
(72) Erfinder: BIENHAUS, Gerhard, D-82407 Wielenbach (DE); SCHUBERT, Ulrich, D-82319 Starnberg (DE); KOLB, Uwe, D-82362 Weilheim (DE); STOLZ, Burkhard, D-82386 Huglfing (DE); PASCH, Manfred, D-82327 Tutzing (DE)
(86) Internationale Anmeldenummer: EP9601368
(87) Internationale Veröffentlichungsnummer: WO96031781

(56) Entgegenhaltungen:
- EP-A- 0 265 244
- EP-A- 0 389 063
- EP-A- 0 687 502
- WO-A-91/15768
- WO-A-93/25912

## Beschreibung

Gegenstand der Erfindung sind ein System zur Freisetzung und Isolierung von Nukleinsäuren und ein Verfahren zur Benutzung dieses Systems.

Nachweisverfahren, die auf der Bestimmung von Nukleinsäuren in einer Probe beruhen, sind in jüngerer Zeit verstärkt mit Interesse bedacht worden. Dies liegt unter anderem in der erreichbaren hohen Spezifität des Nachweises. Hierin sind Nukleinsäurenachweise den Antigennachweisen prinzipiell überlegen. Während Antigene jedoch oft in einer Probe schon relativ zugänglich vorliegen, müssen Nukleinsäuren, insbesondere bei Nachweisen von Organismen, meist in mehreren Schritten zugänglich gemacht werden. Darüber hinaus sind Nukleinsäuren in der Regel in sehr geringen Konzentrationen vorhanden. Insbesondere bei der Isolierung von Nukleinsäuren aus zellhaltigen Proben sind bisher aufwendige Aufreinigungsverfahren bekannt.

Die Sensitivität der auf dem Markt derzeit angebotenen Probenanreicherungs- und Probenvorbereitungssysteme für Nukleinsäuren ist oft nicht hinreichend groß. Bei der Probenvorbereitung mit automatisierten Verfahren ist zudem keine ausreichende Kontaminationssicherheit gegeben, die eine Amplifikation z. B. mittels PCR ermöglichen würde. Die zur Zeit erhältlichen automatischen Probenvorbereitungssysteme benötigen zudem organische Lösungsmittel (Phenol- und/oder Chloroform-Alkoholgemische) zur Gewinnung der Nukleinsäuren.

Die derzeit eingesetzten Verfahren unter Verwendung einer Immobilisierung der Nukleinsäuren benutzen im wesentlichen zwei Prinzipien zur Isolierung der Nukleinsäuren. In einer ersten Möglichkeit werden nukleinsäurehaltige flüssige Proben durch eine Festphasenmatrix gesaugt, wobei die Nukleinsäuren in der Festphasenmatrix festgehalten werden. Dies setzt einen vorherigen Lyseschritt voraus, der in einem getrennten Gefäß durchgeführt wurde. Anschließend werden die Nukleinsäuren durch Durchsaugen einer Elutionsflüssigkeit von der Festphasenmatrix gelöst. Die nukleinsäurehaltige Elutionslösung wird in ein Gefäß zur Weiterbearbeitung abgesaugt. Es hat sich jedoch herausgestellt, daß die derzeit verwendeten Vorrichtungen im Hinblick auf die für die Durchführung einer späteren Amplifikationsreaktion, z. B. PCR, erforderlichen Reinheit nicht ausreichend sind.

Bei einem zweiten Prinzip werden die Nukleinsäuren ausgefällt und mittels einer Zentrifuge separiert. Bei diesem Verfahren ist jedoch ein sogenannter Batch-Betrieb unumgänglich. Bei einem solchen Verfahren wird beispielsweise eine zellhaltige Lösung in einem ersten Reaktionsgefäß mit lysierenden Agenzien behandelt. Anschließend wird die Reaktionsmischung aus dem Gefäß in ein Zentrifugationsröhrchen umpipettiert. Dieses Röhrchen hat einen Einsatz, an welchem die freigesetzten Nukleinsäuren adsorbieren können, während die restliche Flüssigkeit während der Zentrifugation in den unteren Bereich des Röhrchens fließen kann. Zum Waschen der absorbierten Nukleinsäuren wird der Einsatz ein- oder mehrmals mit einer Waschflüssigkeit behandelt. Hierzu muß der Einsatz in ein weiteres Zentrifugationsröhrchen überführt werden, damit nicht Reste der Probenflüssigkeit wieder zurück in den Einsatz gelangen. Im letzten Schritt wird der Einsatz in ein weiteres neues Gefäß eingesetzt. Die Nukleinsäuren werden durch Zentrifugation einer Elutionslösung durch den Einsatz in ein weiteres Gefäß hinein in eine weiterverarbeitungsfähige Lösung überführt. Dieses Verfahren ist jedoch einerseits mit einem hohen Kontaminationsrisiko behaftet und andererseits sind eine Vielzahl von Wechseln der Reaktionsgefäße erforderlich.

In dem Dokumtent EP-A-0 265 244 ist ein Verfahren und eine Vorrichtung beschrieben, bei dem der Nachweis von Nukleinsäuren über eine Bindung an Magnetpartikel und Abscheidung der Magnetpartikel erfolgt. Bei der beschriebenen Vorgehensweie werden Aufheizungs-, Schüttel- und magnetische Abscheidungsschritte verwendet. Hierbei wird die Probe jedoch bereits vor einer Abscheidung von Magnetpartikeln aufgeschlossen. Weiterhin werden die einzelnen Verfahrensschritte in verschiedenen Stationen durchgeführt, so daß ein Transport der Probe durch die einzelnen Stationen notwendig ist.

Weiterhin ist im Stand der Technik das Dokument WO 92/05443 bekannt, in dem eine Apparatur zum Abscheiden von mit Reagenz beladenen Magnetpartikeln beschrieben ist. Das Dokument offenbart eine kombinierte Abscheider-/Schüttlervorrichtung, die mit Mikrotiterplatten arbeitet.

Aufgabe der vorliegenden Erfindung war es, ein System bereitzustellen, bei dem die Nachteile des Standes der Technik vollständig oder zumindest teilweise beseitigt werden. Insbesondere können mit diesem System Nukleinsäuren an einer Festphasenmatrix absorbiert und desorbiert werden, ohne daß für diese Schritte eine Zentrifuge erforderlich wäre.

Ein wesentlicher Aspekt der Erfindung ist eine Aufnahme für ein Probenbearbeitungsgefäß, welches temperiert und in eine Bewegung versetzt werden kann, so daß eine Durchmischung der in dem Probenbearbeitungsfäß befindlichen Substanzen erreicht wird. Zudem verfügt diese Aufnahme über einen Anschluß an ein einen Unterdruck erzeugendes System (z. B. Schlauchpumpe, Kolbenhubpumpe). Außerdem ist es mit der Aufnahme möglich, innerhalb der Probenbearbeitungsgefäße magnetische Partikel abzuscheiden. Wesentliche Vorteile der Erfindung sind der Schutz gegen Kontamination (sowohl von Probe zu Probe als auch von System zu Umwelt) sowie die Möglichkeit, ausreichend viele Proben bearbeitungs Gefäße aufzunehmen, um ein wirtschaftliches Betreiben dieses Systems zu gewährleisten.

Gegenstand der Erfindung ist ebenfalls ein Verfahren nach Anspruch 1 zur Freisetzung und Isolierung oder zum Nachweis von Nukleinsäuren aus biologischen Kompartimenten einer Probe.

Ein weiterer Gegenstand der Erfindung ist ein System zur Freisetzung und Isolierung von Nukleinsäuren aus einer Suspension von biologischen Kompartimenten mit Magnetpartikeln nach Anspruch 5.

Nukleinsäuren im Sinne der vorliegenden Erfindung sind Nukleinsäuren, die in biologischen Kompartimenten vorliegen. Unter biologischen Kompartimenten werden insbesondere Zellen, z. B. viralen oder bakteriellen Ursprungs verstanden. Besonders bevorzugt liegen die Zellen im wesentlichen im vereinzelten Zustand vor. Prinzipiell können auch mehrzellige Kompartimente im Sinne der Erfindung bearbeitet werden. Diese Kompartimente mit ihren Nukleinsäuren liegen zu Beginn des erfindungsgemäßen Verfahrens in einer Probe vor. Bevorzugt ist diese Probe eine Suspension der biologischen Kompartimente in einer Flüssigkeit. Solche Proben können beispielsweise erhalten werden aus Körperflüssigkeiten, z. B. Blut, Speichel oder Urin.

Unter Freisetzung der Nukleinsäuren wird im Sinne der Erfindung der Austritt der Nukleinsäuren aus den biologischen Kompartimenten verstanden. Dieser Austritt kann auf beliebige Weise geschehen. Bevorzugt findet der Austritt durch Zerstörung der die biologischen Kompartimente gegen die Flüssigkeit abgrenzenden Wand statt. Dies kann beispielsweise erreicht werden durch Behandlung der Kompartimente mit zellwandzerstörenden Mitteln, z. B. Proteinase K.

Unter der Isolierung von Nukleinsäuren wird die Abtrennung der Nukleinsäure von anderen Bestandteilen der Probe verstanden. Solche anderen Bestandteile sind beispielsweise die Wände der biologischen Kompartimente, deren Abbauprodukte, weitere Inhaltsstoffe der biologischen Kompartimente sowie Inhaltsstoffe der Flüssigkeit, welche die biologischen Kompartimente in der Probe umgibt. Hierzu gehören beispielsweise Proteine, Inhibitoren für Enzyme, insbesondere Nukleinsäure-abbauende Enzyme, wie Dnase oder RNase. In diesem Sinne kann Isolierung auch als eine Art Reinigung der Nukleinsäuren verstanden werden. Diese Isolierung kann sowohl spezifisch als auch unspezifisch im Hinblick auf weitere in der Probe enthaltenen Nukleinsäuren sein.

Unter einem Nachweis von Nukleinsäuren wird erfindungsgemäß ein Verfahren verstanden, bei welchem die Anwesenheit oder Menge von Nukleinsäuren bestimmt wird. Diese Verfahren können sowohl quantitativ als auch qualitativ vorgenommen werden. Für die Durchführung quantitativer Nachweise wird in der Regel ein Vergleichsversuch mit einer Probe durchgeführt, die eine bekannte Menge der nachzuweisenden Nukleinsäuren enthält. Der Nachweis kann sowohl sequenzspezifisch als sequenzunspezifisch sein. Um die Nachweise spezifisch zu machen, verwendet man in der Regel sogenannte Sonden, die dadurch gekennzeichnet sind, daß sie eine Nukleobasensequenz aufweisen, die mehr oder weniger charakteristisch für die Nukleinsäuren in der Probe ist. Sofern ein spezifischer Nachweis von Nukleinsäuren gewünscht wird, wird eine Sonde eingesetzt, die eine Basensequenz enthält, welche komplementär zu der Basensequenz der nachzuweisenden Nukleinsäure, nicht jedoch zu anderen Nukleinsäuren in der Probe, ist. Sonden können Moleküle sein, die eine direkt oder indirekt nachweisbare Gruppe enthalten. Direkt nachweisbare Gruppen sind beispielsweise radioaktive (³²P) farbige oder fluoreszierende Gruppen oder Metallatome. Indirekt nachweisbare Gruppen sind beispielsweise immunologisch oder enzymatisch wirksame Verbindungen, wie Antikörper, Antigene, Haptene, Enzyme oder enzymatisch aktive Teilenzyme. Diese werden in einer nachfolgenden Reaktion oder Reaktionssequenz detektiert. Besonders bevorzugt sind Haptene, z. B. Digoxygenin oder Biotin. Solche haptenmarkierten Sonden können in einer anschließenden Reaktion mit einem markierten Antikörper gegen das Hapten leicht nachgewiesen werden.

### Beschreibung des Systems:

Gegenstand der Erfindung ist ein System zur Freisetzung und/oder Isolierung von Nukleinsäuren aus einer Suspension von biologischen Kompartimenten, enthaltend die Komponenten
- eine Aufnahmeeinheit (10) für eines oder mehrere Probenbearbeitungsgefäße (A),
- eine Thermostatoreinheit (20) zur Thermostatisierung der Probenbearbeitungsgefäße (A) und darin enthaltener Flüssigkeiten,
- einen Schüttler (30) zum Schütteln der Probenbearbeitungsgefäße (A) und
- einen Abscheider (40) zur magnetischen Abscheidung der Magnetpartikel an einer Wand jedes Probenbearbeitungsgefäßes (A),
- eine Pumpeinheit (50) zur Entfernung von Flüssigkeit aus dem Probenbearbeitungsgefäß (A).

Das System hat eine leicht zu reinigende Oberfläche und schützt den Anwender vor Verbrennungen (z. B. durch einen Kunststoffmantel).

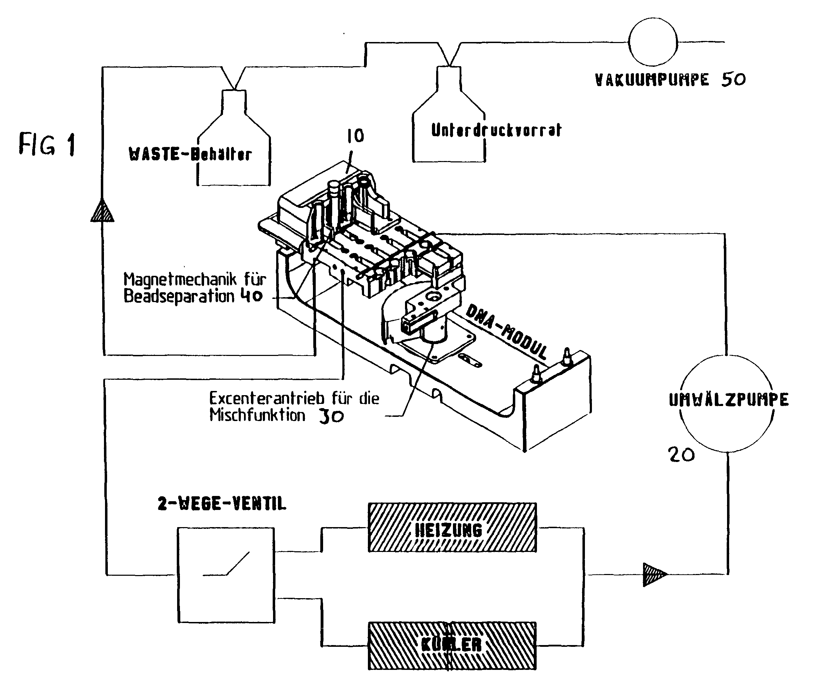

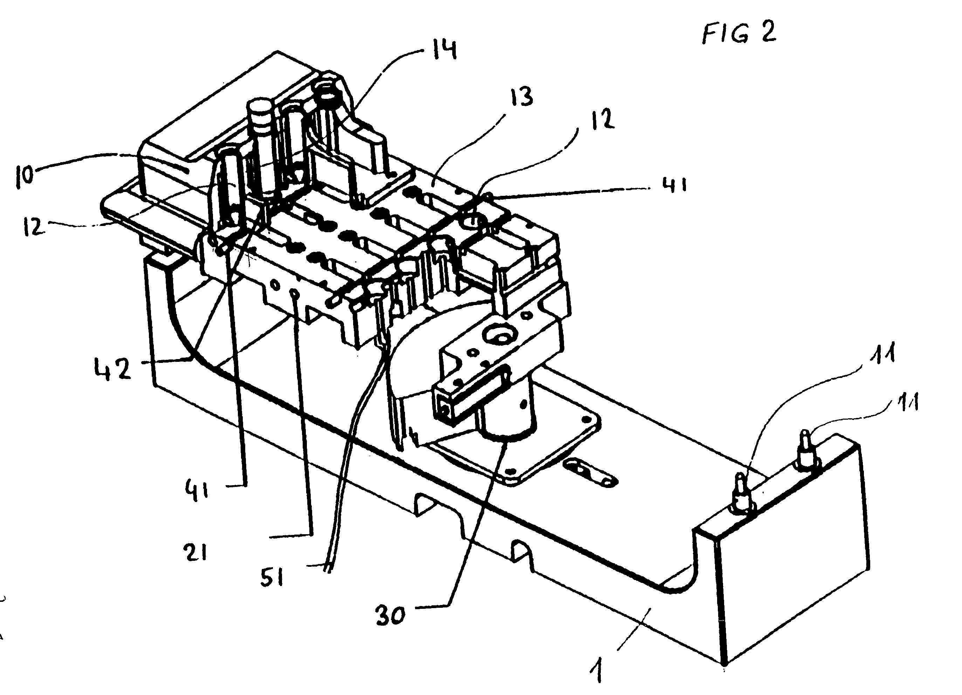

In FIG 1 und 2 ist ein System mit erfindungsgemäßen Einheiten schematisch dargestellt.

Das System besitzt eine Aufnahmeeinheit (10) für die Probenbearbeitungsgefäße. Diese Aufnahmeeinheit enthält mehrere Vertiefungen (12), auch Kavitäten genannt, in welchen die Probengefäße plaziert werden. Die Anordnung der Kavitäten zueinander ist bevorzugt linear oder in Untergruppen linear, die zueinander im rechten Winkel plaziert sind. Der Abstand der Kavitäten entspricht bevorzugt dem Abstand von Mulden einer Mikrotiterplatte, weiter bevorzugt wird das Doppelte des vorgenannten Abstandes. Die Kavitäten sind in ihrer Geometrie den Probengefäßen angepaßt.

Das Probenbearbeitungsgefäß (A) kann prinzipiell jede beliebige Form aufweisen. Solche Probenbearbeitungsgefäße können z. B. die Vertiefungen einer Mikrotiterplatte (z. B. im 96 Wellformat) sein. Bevorzugt handelt es sich jedoch um zylindrische Gefäße, welche eine obere Einlaßöffnung und, besonders bevorzugt, eine untere Auslaßöffnung (A11) besitzen. Ein solches Probenbearbeitungsgefäß kann zur kontaminationsreduzierten Bearbeitung von nukleinsäurehaltigen Proben verwendet werden. Diese Gefäße bestehen in der Regel aus Kunststoffen, z. B. Polypropylen.

Besonders bevorzugt können im Rahmen der vorliegenden Erfindung Gefäße eingesetzt werden, wie sie in den deutschen Gebrauchsmusteranmeldungen Aktenzeichen 29505652.5 und 29505707.6 beschrieben sind.

Die Temperierung der Flüssigkeit in den Probenbearbeitungsgefäßen wird erfindungsgemäß durch eine Thermostatoreinheit (20) vorgenommen. Diese Einheit, welche prinzipiell aus für Thermostate üblichen Baueinheiten besteht, ist vorzugsweise in die Aufnahmeeinheit (10), in welcher die Probenbearbeitungsgefäße positioniert werden können, zumindest teilweise integriert. Die Einheit (10) enthält insbesondere einen Block aus Metall, welcher gute Wärmeleiteigenschaften hat. Dieser ist auf die äußere Form der Probenbearbeitungsgefäße so abgestimmt, daß Probenbearbeitungsgefäße, die sich in der Aufnahme befinden, mit ihrer Wandung möglichst gut an dem Metallblock anliegen, so daß ein effizienter Wärmeübertrag möglich ist. In Abhängigkeit von der in dem Probenbearbeitungsgefäß durchzuführenden Reaktion wird die Temperatur in dem Block erhöht oder erniedrigt. Das Temperaturspektrum erstreckt sich von 4 - 95 °C. Idealerweise wird die Temperaturerhöhung durch mehrere elektrische Heizelemente realisiert, die sich möglichst nahe an dem Probenbearbeitungsgefäß befinden. Die Kühlung der Flüssigkeit im Probenbearbeitungsgefäß wird durch Peltierelemente realisiert, die ebenfalls möglichst nahe an den Probenbearbeitungsgefäßen plaziert sind. Eine Temperaturregelung erfolgt bevorzugt über einen Fuzzy-Regler oder einen PIDT-Regler. In die Thermostatoreinheit sind Thermofühler in ausreichender Zahl an repräsentativen Stellen plaziert.

Eine weitere Ausführungsform der Thermostatoreinheit (20) besitzt einen von Flüssigkeit durchströmten Block. Als Temperiermedium dienen Flüssigkeiten; meist Wasser oder wässrige Salzlösungen. Die Temperierflüssigkeit wird bevorzugt über flexible Schläuche von einer Heizung bzw. Kühlung mittels einer Umwälzpumpe direkt in Öffnungen (21) im Block (10) eingespeist. Das Fassungsvermögen des Temperierreservoirs (in Fig. 1 bezeichnet mit Heizung und Kühler) außerhalb des DNA-Moduls ist um ein vielfaches größer als das Totvolumen des DNA-Moduls damit beim Umschalten des 2-Wege-Ventils diese Störgröße minimiert wird. Die Heizung und der Kühler temperieren im Vorlauf und werden bei Bedarf getaktet. Eine Regelung schaltet das Ventil, Heizung und Kühlung, zusammen mit dem geeigneten Volumenstrom der Umwälzpumpe werden die gewünschten Heiz- und Kühlraten erreicht. Als Thermostatoreinheit (20) ist bei dieser Ausführungsform die Kombination von flüssigkeitsdurchströmten Block, Umwälzpumpe, Heizung, Kühler und 2-Wege-Ventil zu verstehen. Als DNA-Modul wird die Vorrichtung umfassend Aufnahmeeinheit für Probenbearbeitungsgefäß, Schüttler und Abscheider bezeichnet.

Das System ermöglicht das Arbeiten mit magnetischen Partikeln (Beads). Unter Magnetpartikeln werden Partikel verstanden, welche durch einen Magneten in eine bestimmte Richtung transportiert werden können. Hierzu gehören beispielsweise ferromagnetische oder superparamagnetische Materialien. Besonders bevorzugt im Sinne der Erfindung sind ferromagnetische Materialien. Partikel sind feste Materialien mit einem geringen Durchmesser. Im Sinne der Erfindung sind besonders Partikel geeignet, die eine durchschnittliche Korngröße von mehr als 2, 8 µm, jedoch weniger als 200 µm haben. Besonders bevorzugt weisen sie eine durchschnittliche Korngröße zwischen 10 und 15 µm auf. Bevorzugt ist die Korngrößenverteilung homogen. Diese Partikel sind an ihrer Oberfläche so modifiziert, daß sie die biologischen Kompartimente binden können. Hierfür geeignete Magnetpartikel sind die bekannten und käuflichen Latexmagnetpartikel, an welche z. B. Antikörper gebunden sein können. Zur Bindung der biologischen Kompartimente an die Magnetpartikel werden insbesondere Antikörper verwendet, welche gegen Oberflächenantigene der biologischen Kompartimente gerichtet sind. Derartige Magnetpartikel sind ebenfalls kommerziell erhältlich.

Des weiteren können glasmagnetische Pigmente eingesetzt werden, an deren Oberflächen Nukleinsäuren binden. Solche glasmagnetischen Pigmente sind aus der deutschen Patentanmeldung 19537985.3 bekannt.

Damit das erfindungsemäße Verfahren erfolgreich durchgeführt werden kann, ist es notwendig, die Magnetpartikel für bestimmte Reaktionsschritte an der Innenwand des Probenbearbeitungsgefäßes zu binden und mit einem nachfolgenden Schritt wieder in Suspension zu bringen. Im Sinne der Erfindung, als besonders bevorzugt, wird für die Positionierung der Magneten ein Abscheider (40) mit einem oder mehreren an dem Abscheider befestigten Permanentmagneten oder Elektromagneten an das Probenbearbeitungsgefäß herangefahren. Die für eine effiziente Abscheidung erforderliche Entfernung des Magneten vom Probenbearbeitungsgefäß hängt stark von der Größe des durch den Magneten erzielbaren Magnetfeldes und der Größe und Magnetisierbarkeit der Magnetpartikel ab. Außerdem hat die Art der später folgenden Bearbeitungsschritte (z. B. mechanische Belastung der Magneten) einen Einfluß auf die zu verwendende Magnetfeldstärke. Sofern es sich um einen Permanentmagneten handelt, wird dieser aus einer Position, die nicht für eine Abscheidung der Magnetpartikel ausreicht, in die Nähe des Gefäßes gebracht, so daß die Magnetpartikel an der Gefäßwand festgehalten werden. Für den Fall der Verwendung eines Elektromagneten wird dieser eingeschaltet und solange im eingeschalteten Zustand belassen, bis eine Bearbeitung der festgehaltenen biologischen Kompartimente abgeschlossen ist.

Unter der Positionierung eines Magneten in der Nähe des Gefäßes soll auch der Fall verstanden werden, daß das Gefäß in die Nähe des Magneten gebracht wird. Letztendlich kommt es also nur auf die Relativbewegung des Magneten zum Gefäß an.

Der Abscheider (40) weist bevorzugt einen Magneten auf, welcher auf einer vorbestimmten Bahn, z. B. über Schienen oder, bevorzugt durch Bewegung des Magneten auf einer Kreisbahn, z. B. um eine neben dem Probengefäß liegende Achse, auf das Probenbearbeitungsgefäß hin beweglich ist. Der Abscheider beinhaltet außerdem einen Motor, welcher sowohl die Bewegung des Magneten auf das Probenbearbeitungsgefäß zu als auch dessen Wegbewegung realisieren kann.

In einer weiteren Ausführung weist der Abscheider(40) eine Zahnstange auf, die über einen Gleichstrommotor oder alternativ über einen Schrittmotor linear verfahren werden kann. Rechtwinklig angeordnet zu der Zahnstange befindet sich eine Haltevorrichtung für die Magnete, in diesem Fall Permanentmagnete. Die Endpositionen der Magnetbewegung können über Lichtschranken detektiert werden. Bevorzugt wird jedem Probenbearbeitungsgefäß ein Magnet zugeordnet, der mit seiner Stirnseite an dieses herangeführt wird. Es ist jedoch auch möglich, an jedes Probenbearbeitungsgefäß 2 Magnete heranzuführen. Dabei wird bevorzugt jeweils 1 Magnet an 2 Gefäße herangeführt, so daß für n Gefäße lediglich n+1 Magnete einzusetzen sind.

Zur Abscheidung müssen die Magnete möglichst nah an die Probenbearbeitungsgefäße herangeführt werden, um eine hohe Abscheiderate der magnetischen Partikel zu erzielen. Wichtig ist auch ein ausreichender Verfahrweg der Magnete zwischen den Positionen. In Abhängigkeit vom verwendeten Magnetmaterial und der Magnetgeometrie müssen die Magnete bis zu 40 mm vom Probenbearbeitungsgefäß entfernt werden um eine ungewollte Abschediung zu verhindern. Wichtig für die Funktion des Instrumentes ist es, daß die magnetischen Kräfte nur dann auf die Partikel im Probenbearbeitungsgefäß wirken, wenn dies gewünscht ist. Somit ist der Magnet in der inaktiven Position so weit vom Gefäß zu verfahren, daß das Magnetfeld keine nennenswerte Wirkung mehr auf die Bewegung der Partikel hat.

Dies kann auch so realisiert werden, daß der Abstand zwischen Magnet und Probenbearbeitungsgefäß nicht verändert wird. Die Magnetfelder können jedoch durch ein µ-Metall, welches zwischen Gefäß und Magnet bewegt wird, unterbrochen werden.

Eine weitere mögliche Anordnung der Magnete erfolgt auf einer drehbaren, von einem Gleichstrommotor angetriebenen Welle. Mit dieser Ausführungsform ist es möglich, die Magnete auf einer Kreisbahn zu bewegen. Die Magnete selbst können auf dieser Welle beliebig angeordnet werden, so daß bei Bewegung der Welle ein Heranführen oder Wegbewegen der Magnete an bzw. von den Probenbearbeitungsgefäßen erfolgt. Darüber hinaus ist eine Antrieb vorteilhaft, bei der auf gegenüberliegenden Seiten des Systems je 1 Welle mit einer Anzahl Magneten angeordnet ist. Die Wellen werden über einen Zahnriemen von einem Motor angetrieben. Bei dieser Anordnung bewegen sich je 2 Magnete synchron auf die Stirnseiten der Probenbearbeitungsgefäße zu.

Magneten für die vorliegende Erfindung weisen bevorzugt eine Masse zwischen 0,5 und 5 g, besonders bevorzugt zwischen 1 und 4 g auf. Die äußeren Abmessungen, die im Prototyp verwendet wurden, betragen 10 mm x 10 mm x 3 mm. Als geeignetes Material für einen Permanentmagneten haben sich seltene Erdmaterialien (z. B. NeFeB, VACODYM 370 HR) mit einem optimalen BH-Maximum bei kleinsten Abmessungen erwiesen. Für eine effiziente Abscheidung ist es vorteilhaft, den Gradienten des Magnetfeldes besonders ausgeprägt zu dimensionieren. Aus diesem Grund sollte auch die Positionierung des Magneten möglichst nah an dem Gefäß erfolgen. Bevorzugt werden für die Probenbearbeitungsgefäße Materialien mit einer möglichst geringen Dämpfung des Magnetfeldes eingesetzt, z. B. Polypropylen.

An der Baueinheit zur Aufnahme der Probenbearbeitungsgefäße sind an der Unterseite der Kavität Schläuche (51) befestigt, die wiederum mit einem, einen Unterdruck erzeugenden System, verbunden sind. Jeder Kavität ist ein Schlauch zugeordnet. Da im Bodenbereich der Probenbearbeitungsgefäße Öffnungen vorgesehen sind, kann durch Erzeugen eines Unterdruckes der Inhalt der Probenbearbeitungsgefäße abgesaugt und in den Abfall (Waste) überführt werden. In der Ausführung gemäß FIG 2 besitzt jede Kavität eine Dichtung, die beim Absaugen des Wastes verhindert, daß Luft zwischen Probenbearbeitungsgefäß und Inlet (14) angesaugt wird.

Das Unterdrucksystem besteht im wesentlichen aus einer Kolbenhubpumpe (50), die über ein Schlauchsystem mit den Kavitäten verbunden ist. Dazwischen ist ein Waste-Behälter geschaltet, der die Flüssigkeit aufnimmt. Die Flüssigkeit, welche aus den Probenbearbeitungsgefäßen abgesaugt wird, ist Abfall. Des weiteren ist zwischen jedes Probenbearbeitungsgefäß und dem Waste-Behälter ein Ventil geschaltet. Dieses Ventil ermöglicht das Schalten des Unterdrucks zu jeder Kavität, wenn das System von der Pumpe über die Abfallflasche (Waste-Behälter) bis hin zum Ventil permanent evakuiert ist. Es ist erfindungsgemäß sowohl ein paralleles als auch ein sequentielles Absaugen der Probenbearbeitungsgefäße möglich.

In einer anderen Ausführungsform werden Kolbenhubpumpe und Ventile durch eine Schlauchpumpe ersetzt. Die Abfallflasche ist in diesem Fall nicht permanent evakuiert, sie befindet sich im Fluidstrom hinter den Kavitäten und der Schlauchpumpe. Diese Anordnung erlaubt nur ein paralleles Absaugen der Kavitäten. Es können auch mehrere Schlauchpumpen eingesetzt werden, die dann eine bestimmte Anzahl von Kavitäten bedienen. Damit ist ein teilweise sequentielles Arbeiten möglich.

Die Bewegung der Probenbearbeitungsgefäße erfolgt bevorzugt in horizontaler Richtung. Besonders bevorzugt wird die Aufnahmeeinheit (10) bewegt, welche Vertiefungen (12) mit jeweils einem ProbengefäB enthält, so daß alle darin befindlichen Probengefäße geschüttelt werden. Über Schwingungsdämpfer (11) wird die Übertragung der Bewegung auf das Gesamtgerät reduziert. Im Sinne der Erfindung bevorzugt ist die Verwendung eines Schüttlers (30), der die Bewegung der Probenbearbeitungsgefäße (A) durchführt. Diese Einheit kann jede prinzipiell zum Mischen von Flüssigkeiten in einem Gefäß geeignete mechanische Einrichtung sein. Ein bevorzugtes Beispiel einer solchen Einheit ist im Folgenden beschrieben.

Ein Schrittmotor mit einem Excenter und einer Ausgleichsmasse bewegen von einem festen Rahmen (1) die über Schwingungsdämpfer auf diesen Rahmen aufgesetzte Aufnahmeeinheit (10) in einer kreisförmigen exzentrischen Bahn, fester Amplitude und variabler Frequenz. Die bevorzugte Amplitude A ist ≤ 1,5 mm, die bevorzugte Frequenz (f) ist größer als 1 Hz und kleiner als 50 Hz. Die Misch- bzw. Resuspensionsdauer beträgt je nach physikalischen Eigenschaften des Probenmaterials zwischen 5 und 30 s. Durch Austausch des Excenters ist es möglich, die Amplitude zu variieren.

Die Kombination des erfindungsgemäßen Systems mit einem Pipettierautomaten ist als solche nicht naheliegend, da hierfür das Vorsehen einer definierten Positionierung der Probengefäße vor und während der Pipettierschritte erforderlich ist. Das Schütteln der Gefäße führt sonst dazu, daß sich die Gefäße nach jedem Schüttelvorgang an einer anderen Position befinden. Sofern die Position der Gefäße während des Pipettiervorganges nicht genau spezifiziert ist, besteht die Gefahr, daß der Pipettiervorgang nicht ordnungsgemäß durchgeführt werden kann. Deshalb wird dafür gesorgt, daß sich das Gefäß nach dem Schütteln in einer definierten, sogenannten Home-Position befindet, in welcher eine Pipettierung oder andere Vorgänge stattfinden können.

Vorteilhaft ist der Einsatz eines Schrittmotors gegnüber dem Einsatz eines Gleichstrommotors, um eine definierte Home-Position zu gewährleisten. Die Home-Position kann vorteilhaft mit einer Lichtschranke detektiert werden.

In Bezug auf die konstruktive Ausführung bestehen noch die folgenden non-invasiven alternativen Möglichkeiten, die aber alle in der Konstruktion aufwendiger sind (Varianten 1 und 2) oder in den Mischschritten (3.) länger dauern:
1. Eine Kombination von eins, zwei oder drei linearen Antrieben für die Aufnahmeeinheit in der Ebene bzw. im Raum (X-, Y-, Z-Achse) zur Erzeugung von z. B. Lissajous-Figuren.
2. Taumeln, durch Neigen des Rahmens (1) um einen bestimmten Winkel und Gegenstellen der Aufnahmeeinheit (10).
3. Magnetrührer
4. Schwenken oder Klopfen des DNA-Moduls.

Die Kopplung der Komponenten des Systems ist einerseits funktionell, z. B. durch Integration der Magneten in die Einheit 10. Außerdem sind die Systemkomponenten zeitlich gekoppelt. Zum Beispiel wird der Betrieb der Einheiten für die gewünschte Anwendung in geeigneter Abfolge gesteuert, was beispielsweise durch ein Computerprogramm oder durch Initiation der einzelnen Teilschritte durch den Anwender erfolgen kann.

### Beschreibung des Verfahrens:

### Variante A:

In einem ersten Schritt wird die Probe in einem Probenbearbeitungsgefäß zusammen mit magnetischen Partikeln (beads), welche die biologischen Kompartimente binden können, unter Schütteln des Probenbearbeitungsgefäßes inkubiert.

Die Inkubation der Proben mit den Magnetpartikeln kann auf beliebige Weise gestaltet werden. Erforderlich ist, daß sowohl die Probe als auch die magnetischen Partikel in das Probenbearbeitungsgefäß eingebracht werden. Sowohl die Art der Einbringung als auch deren Reihenfolge ist prinzipiell ohne größere Bedeutung für das erfindungsgemäße Verfahren. Bevorzugt jedoch werden die magnetischen Partikel in Form einer Suspension mit einem bekannten Gehalt magnetischer Partikel in das Probenbearbeitungsgefäß pipettiert. Entweder anschließend oder vorher wird die Probe in das Probenbearbeitungsgefäß einpipettiert.

Die Inkubation wird solange unter geeigneten Bedingungen vorgenommen, bis eine ausreichende Menge an biologischen Kompartimenten an die Magnetpartikel gebunden sind. Es wird sich hierbei im Regelfall um einen Zeitraum zwischen 1 min und 10 min handeln. Das Probenbearbeitungsgefäß ist hierbei bevorzugt auf geeignete Weise, z. B. mittels eines Dèckels oder/und eines Ventils verschlossen.

Ein wesentliches Merkmal der Erfindung ist, daß das in dem Probenbearbeitungsgefäß befindliche Gemisch während der Inkubation geschüttelt wird. Es kann sich hierbei um ein Intervallschütteln handeln. Das Schütteln kann jedoch auch während der gesamten Inkubationszeit oder nur Teilen davon durchgeführt werden. Das Schütteln dient dazu, eine ausreichende Mischung der biologischen Kompartimente und der Magnetpartikel in der Flüssigkeit zu erreichen, insbesondere die Suspension bzw. Resuspension der Beads und die Beschleunigung der Diffussion. Hierdurch wird die für die Bindung der biologischen Kompartimente an die Magnetpartikel erforderliche Zeit der Inkubation reduziert.

Im Anschluß an die Inkubation und Bindung der Kompartimente an die Magnetpartikel werden die biologischen Kompartimente von der sie umgebenden Flüssigkeit der Probe entfernt. Hierzu hat es sich als zweckmäßig erwiesen, die Magnetpartikel mit den daran gebundenen biologischen Kompartimenten durch Positionierung eines Magneten in der Nähe des Probenbearbeitungsgefäßes zu sammeln. Hierdurch werden bevorzugt die Magnetpartikel mit den biologischen Kompartimenten an der Gefäßwand festgehalten. Die Abscheidung der Beads erfolgt also in der Regel an der Innenwand des Probenbearbeitunsggefäßes oder einem Teil davon, welche unter der Flüssigkeitsoberfläche der Probe befindlich ist.

Anschließend wird die die biologischen Kompartimente umgebende Flüssigkeit aus dem Probenbearbeitungsgefäß entfernt. Dies geschieht unter Bedingungen, bei denen die Magnetpartikel an der Gefäßwand zurückbleiben. Die Art der Entfernung hängt von der Art des Probenbearbeitungsgefäßes ab. Sie kann z. B. abpipettiert werden. In einer bevorzugten Ausführungsform jedoch, bei der das Probenbearbeitungsgefäß eine untere Auslaßöffnung aufweist, wird die Flüssigkeit durch diese einfach abgesaugt. Diese Art der Entfernung hält die mechanische Belastung der Magnetpartikel gering und vermeidet somit die Ablösung der Magnetpartikel von der Gefäßwand.

Ein besonders wichtiger Schritt ist die Resuspension der an der Gefäßwand zurückgehaltenen Magnetpartikel in einer zugegebenen zweiten Flüssigkeit. Hierzu wird der Magnet aus der Nähe des Gefäßes entfernt, so daß die Magnetpartikel nicht mehr durch den Magneten an der Gefäßwand festgehalten werden. Wie oben beschrieben ist es auch möglich, das Gefäß aus der Nähe des Magneten zu entfernen. Gemäß der vorliegenden Erfindung hat sich das einfache Entfernen des Magneten als nicht ausreichend für eine Resuspension erwiesen, wenn nicht das Gefäß ergänzend, bevorzugt gleichzeitig geschüttelt wird. Dieses Schütteln wird mit dem Schüttler (30) durchgeführt. Es bewirkt eine gleichmäßige Verteilung der Magnetpartikel in der zweiten Flüssigkeit. Diese zweite Flüssigkeit kann vor Entfernen des Magneten, jedoch auch erst nach Entfernen des Magneten in das Probenbearbeitungsgefäß eingefüllt werden, z. B. durch Einpipettieren.

Das erfindungsgemäße Verfahren kann auch zur weiteren Aufreinigung von biologischen Kompartimenten verwendet werden. Hierzu wird eine Suspension der Magnetpartikel, welche die biologischen Kompartimente gebunden enthalten, in einem Probenbearbeitungsgefäß so in Relation zu einem Magneten positioniert, daß die Magnetpartikel mit den biologischen Kompartimenten an der Gefäßwand festgehalten werden, anschließend die Flüssigkeit, welche die biologischen Kompartimente enthielt, aus dem Gefäß entfernt wird und anschließend die Magnetpartikel in einer zweiten Flüssigkeit, hier einer Waschflüssigkeit, durch Entfernen des Magneten aus der Nähe des Gefäßes, so daß die Magnetpartikel nicht mehr durch den Magneten an der Gefäßwand festgehalten werden, und gleichzeitig Schütteln des Gefäßes resuspendiert. Dieser Waschvorgang kann beliebig wiederholt werden, bis eine ausreichende Reinheit der biologischen Kompartimente erreicht ist.

Als weiterer Schritt des erfindungsgemäßen Verfahrens ist anschließend der Aufschluß (Lyse) der biologischen Kompartimente vorgesehen. Verfahren zum Aufschluß biologischer Kompartimente sind dem Fachmann ebenso bekannt, wie die spezifischen Bedingungen für bestimmte Arten von Kompartimenten, z. B. Zellen. Beispielsweise werden für den Aufschluß von Bakterien die biologischen Kompartimente mit einer Mischung von Proteinase K versetzt und für eine bestimmte Zeit inkubiert, die für das Aufbrechen bzw. den teilweisen oder vollständigen Verdau der Zellwände unter Freisetzung der in den biologischen Kompartimenten enthaltenen Nukleinsäuren inkubiert wird. Dabei wird bevorzugt bei Temperaturen über Raumtemperatur, besonders bevorzugt zwischen 70 und 95 °C gearbeitet. Die Mischung, welche durch den Aufschluß der Zellen erzeugt wird, wird im Folgenden auch als Aufschlußmischung bezeichnet. Die Inkubation wird vorzugsweise über eine Zeit von 5 bis 20, besonders bevorzugt zwischen 10 und 15 Minuten durchgeführt.

Insbesondere, wenn der Aufschluß der Zellen bei Raumtemperatur oder geringfügig erhöhter Temperatur stattgefunden hat, ist es bevorzugt, die Aufschlußmischung anschließend auf höhere Temperaturen zu erhitzen, beispielsweise auf 70 °C, oder, bei potentiell infektiösen Proben, auf 95 °C. Hierbei kann gewünschtenfalls auch das Lysereagenz, sollte es bei weiteren Schritten stören, inaktiviert werden.

Anschließend wird die Aufschlußmischung abgekühlt, und zwar unter Bedingungen, die abhängig sind vom Zweck des erfindungsgemäßen Verfahrens. Soll eine Isolierung der Nukleinsäuren an einer festen Phase stattfinden, werden Bedingungen eingestellt, bei denen die Nukleinsäure an diese feste Phase binden können. Ein geeignetes Verfahren zur Bindung von Nukleinsäuren ist die Inkubation der freigesetzten Nukleinsäuren mit Glasoberflächen unter Anwesenheit chaotroper Salze. Ein solches Verfahren ist beispielsweise beschrieben in EP-A-0 389 063. Hierbei werden die Nukleinsäuren in unspezifischer Weise an die Glasoberfläche gebunden, während andere Bestandteile der biologischen Kompartimente sowie der Aufschlußreagenzien nicht oder nur unwesentlich an die Glasoberfläche gebunden werden. Bevorzugt wird anschließend die Flüssigkeit, welche die übrigen Bestandteile enthält, aus dem Probenbearbeitungsgefäß entnommen, z. B. abgesaugt, während die Glasoberfläche mit den daran gebundenen Nukleinsäuren im Probenbearbeitungsgefäß verbleiben kann. In einer bevorzugten Ausführungsform wird eine feste Phase in Form eines Glasfaservlieses in das Probenbearbeitungsgefäß eingeführt und mit der Mischung inkubiert. Hierdurch werden die Nukleinsäuren an der Glasfaser immobilisiert und können auf einfache Weise mit dem Glasfaservlies aus dem Probenbearbeitungsgefäß entnommen werden.

Für den Fall, daß die Nukleinsäuren nach ihrer Freisetzung nachgewiesen werden sollen, werden diese mit einer Sonde hybridisiert. Bei dieser Sonde handelt es sich, wie oben beschrieben, um ein Molekül, welches eine zu der nachzuweisenden Nukleinsäure oder einen Teil davon komplementären Basensequenz aufweist. In einem bevorzugten Falle handelt es sich um ein Oligonukleotid, welches mit einer nachweisbaren Gruppe markiert ist. Die Abkühlung der Reaktionsmischung findet daher unter Bedingungen statt, bei denen eine Hybridisierung der nachzuweisenden Nukleinsäure mit der Nukleinsäuresonde stattfindet. Diese Temperaturen sind einem Fachmann bekannt. In einer anderen Ausführungsform als Verfahren zum Nachweis von Nukleinsäuren findet eine Hybridisierung zwischen der nachzuweisenden Nukleinsäure und einer Festphasen-gebundenen Nukleinsäuresonde statt. Hierbei kann die Sonde an eine beliebige Festphase, solange sie nur von der übrigen Reaktionsmischung abtrennbar ist, verwendet werden, z. B. Mikrotiterplatten-Kavitäten oder die Innenwand des Probenbearbeitungsgefäßes. Verfahren zur Immobilisierung von Nukleinsäuresonden, insbesondere der sogenannten Fangsonden, sind dem Fachmann bekannt, z. B. aus EP-A-0 523 557.

Im allgemeinen wird sich an die Abkühlung der Mischung eine Abtrennung der zu isolierenden bzw. nachzuweisenden Nukleinsäuren von der sie umgebenden Flüssigkeit, welche ggf. noch Reste der Aufschlußmischung und evtl. der für die Bindung der Nukleinsäuren an eine feste Phase benutzten Reagenzien enthält, anschließen. Hierzu kann, je nach Art der verwendeten festen Phase, eine Filtration oder eine Entfernung der festen Phase aus dem Probenbearbeitungsgefäß oder Abpipettieren der Flüssigkeit aus dem Probenbearbeitungsgefäß vorgenommen werden.

Die gebundenen Nukleinsäuren stehen anschließend entweder zur Aufhebung ihrer Bindung an die feste Phase oder ihren direkten Nachweis in üblichen, dem Fachmann bekannten Verfahren zum Nachweis von Nukleinsäurensequenzen oder einer Markierung zur Verfügung.

### Variante B (nicht gemäß Anspruch 1)

In einem ersten Schritt wird die Probe in ein Probenbearbeitungsgefäß zusammen mit Lyse-Reagenz, glasmagnetischen Partikeln (beads), welche die in den biologischen Kompartimenten enthaltenen Nukleinsäuren binden können, pipettiert und das Probenbearbeitungsgefäßes anschließend verschlossen und geschüttelt. Erforderlich ist, daß sowohl die Probe, das Lyse-Reagenz als auch die glasmagnetischen Partikel in das Probenbearbeitungsgefäß eingebracht werden. Sowohl die Art der Einbringung als auch deren Reihenfolge ist prinzipiell ohne größere Bedeutung für das erfindungsgemäße Verfahren. Bevorzugt jedoch werden die glasmagnetischen Partikel in Form einer Suspension mit einem bekannten Gehalt glasmagnetischer Partikel in das Probenbearbeitungsgefäß pipettiert. Entweder anschließend oder vorher wird die Probe in das Probenbearbeitungsgefäß einpipettiert. Wesentlich ist, daß Probe, glasmagnetische Partikel und Lyse-Reagenz durch Schütteln homogen durchmischt werden.

Als weiterer Schritt des erfindungsgemäßen Verfahrens ist anschließend der Aufschluß (Lyse) der biologischen Kompartimente vorgesehen. Verfahren zum Aufschluß biologischer Kompartimente sind dem Fachmann ebenso bekannt, wie die spezifischen Bedingungen für bestimmte Arten von Kompartimenten, z. B. Zellen. Beispielsweise werden für den Aufschluß von Bakterien die biologischen Kompartimente mit einer Mischung von Proteinase K versetzt und für eine bestimmte Zeit inkubiert, die für das Aufbrechen bzw. den teilweisen oder vollständigen Verdau der Zellwände unter Freisetzung der in den biologischen Kompartimenten enthaltenen Nukleinsäuren inkubiert wird. Dabei wird bevorzugt bei Temperaturen über Raumtemperatur, besonders bevorzugt zwischen 70 und 95 °C gearbeitet. Die Mischung, welche durch den Aufschluß der Zellen erzeugt wird, wird im Folgenden auch als Aufschlußmischung bezeichnet. Die Inkubation wird vorzugsweise über eine Zeit von 5 bis 20, besonders bevorzugt zwischen 10 und 15 Minuten durchgeführt.

Insbesondere, wenn der Aufschluß der Zellen bei Raumtemperatur oder geringfügig erhöhter Temperatur stattgefunden hat, ist es bevorzugt, die Aufschlußmischung anschließend auf höhere Temperaturen zu erhitzen, beispielsweise auf 70 °C, oder, bei potentiell infektiösen Proben, auf 95 °C. Hierbei kann gewünschtenfalls auch das Lysereagenz, sollte es bei weiteren Schritten stören, inaktiviert werden.

Ein wesentliches Merkmal der Erfindung ist, daß das in dem Probenbearbeitungsgefäß befindliche Gemisch während der Inkubation geschüttelt wird. Es kann sich hierbei um ein Intervallschütteln handeln. Das Schütteln kann jedoch auch während der gesamten Inkubationszeit oder nur Teilen davon durchgeführt werden. Das Schütteln dient dazu, eine ausreichende Mischung der biologischen Kompartimente und der Magnetpartikel in der Flüssigkeit zu erreichen, insbesondere die Suspension bzw. Resuspension der Beads und die Beschleunigung der Diffussion. Hierdurch wird die für die Bindung der biologischen Kompartimente an die Magnetpartikel erforderliche Zeit der Inkubation reduziert.

Anschließend wird die Aufschlußmischung abgekühlt, und zwar unter Bedingungen, die abhängig sind vom Zweck des erfindungsgemäßen Verfahrens. Die aus dem biologischen Kompartiment herausgelösten Nukleinsäuren sollen nun unspezifisch an der Oberfläche der glasmagnetischen Partikel binden. Um eine Verbesserung der Bindungseigenschaften zu erzielen, wird der Aufschlußmischung nach der Lyse noch i-Propanol oder Ethanol zugegeben, in Abhängigkeit von der Art des biologischen Kompartiments. Anschließend erfolgt eine weitere Durchmischung des Ansatzes durch Schütteln des Probenbearbeitungsgefäßes. Die Nukleinsäuren werden nun unspezifisch an der Festphasenoberfläche gebunden, wobei andere Bestandteile des biologischen Kompartiments und Aufschlußreagenzien nicht oder nur in unwesentlichen Bestandteilen an die Glasoberfläche adsorbieren.

Nachdem diese Festphasenbindung abgeschlossen ist, wird durch Aktivierung der Magnetfelder das glasmagnetische Pigment mit der gebundenen Nukleinsäure an die Innenwand des Probenbearbeitungsgefäßes abgeschieden und der Überstand aus dem Gefäß entnommen. In einer bevorzugten Ausführungsform jedoch, bei der das Probenbearbeitungsgefäß eine untere Auslaßöffnung aufweist, wird die Flüssigkeit durch diese einfach abgesaugt. Diese Art der Entfernung hält die mechanische Belastung der Magnetpartikel gering und vermeidet somit die Ablösung der Magnetpartikel von der Gefäßwand.

In einem nächsten Bearbeitungsschritt werden die glasmagnetischen Partikel in einer Waschflüssigkeit resuspendiert. Hierzu wird der Magnet aus der Nähe des Gefäßes entfernt, so daß die Magnetpartikel nicht mehr durch den Magneten an der Gefäßwand festgehalten werden. Wie oben beschrieben ist es auch möglich, das Gefäß aus der Nähe des Magneten zu entfernen. Gemäß der vorliegenden Erfindung hat sich das einfache Entfernen des Magneten als nicht ausreichend für eine Resuspension erwiesen, wenn nicht das Gefäß ergänzend, bevorzugt gleichzeitig geschüttelt wird. Dieses Schütteln wird wiederum durch den Schüttler (30) durchgeführt, was eine gleichmäßige Verteilung der Magnetpartikel in der Waschflüssigkeit bewirkt. Diese Waschflüssigkeit kann vor Entfernen des Magneten, jedoch auch erst nach Entfernen des Magneten in das Probenbearbeitungsgefäß eingefüllt werden, z. B. durch Einpipettieren. Der Waschschritt kann beliebig wiederholt werden bis eine ausreichende Reinheit der isolierten Nukleinsäuren erreicht ist.

Die gebundenen Nukleinsäuren stehen anschließend entweder zur Aufhebung ihrer Bindung an die feste Phase oder ihren direkten Nachweis in üblichen, dem Fachmann bekannten Verfahren zum Nachweis von Nukleinsäurensequenzen oder einer Markierung zur Verfügung.

Das erfindungsgemäße Verfahren benutzt also eine Kombination von Bearbeitungsschritten, welche eine Aufnahmeeinheit (10) zur Aufnahme eines oder mehrerer Probenbearbeitungsgefäße, eine Thermostatoreinheit (20) zur Thermostatisierung der Probenbearbeitungsgefäße und darin enthaltener Flüssigkeiten, einen Schüttler (30) zum Schütteln der Probenbearbeitungsgefäße und eine Einheit (40) zur magnetischen Abscheidung der Magnetpartikel an eine Wand jedes Probenbearbeitungsgefäßes verwenden. Überraschenderweise lassen sich diese Verfahrensschritte und Einheiten in einem einzigen Reaktionsblock ausführen. Als Reaktionsblock wird hiermit eine Vorrichtung verstanden, welche die Einheiten 10, 20, 30 und 40 teilweise oder vollständig in aufeinander abgestimmter Kopplung enthält. Auf erfindungsgemäße Weise gelingt es auf einfache Weise einen Vorgang, welcher bisher eine Vielzahl manueller Arbeitsschritte voraussetzte, in einem einzigen Gerät ablaufen zu lassen. Insbesondere hat es sich erwiesen, daß die erfindungsgemäßen Reaktionsblocks besonders effektiv sind. Verfahren zur Freisetzung und Isolierung von Nukleinsäuren können mit ihnen schneller durchgeführt werden als bisher. Es ist darüber hinaus möglich, während der genannten Schritte die Nukleinsäure nicht aus dem Gefäß zu entfernen. Dies stellt sowohl im Hinblick auf den Zeitaufwand, als auch auf die Vermeidung von Kontaminationen einen erheblichen Fortschritt gegenüber dem Stand der Technik dar. Üblicherweise wurden bisher nämlich Abkühlungen von Suspensionen durch manuelle Entnahme eines Probenbearbeitungsgefäßes aus dem Gerät und Eintauchen des Gefäßes in ein Kühlbad durchgeführt. Ein solches Vorgehen hat sich als für die Zukunft in der Routinediagnostik nicht ausreichend geeignet erwiesen.

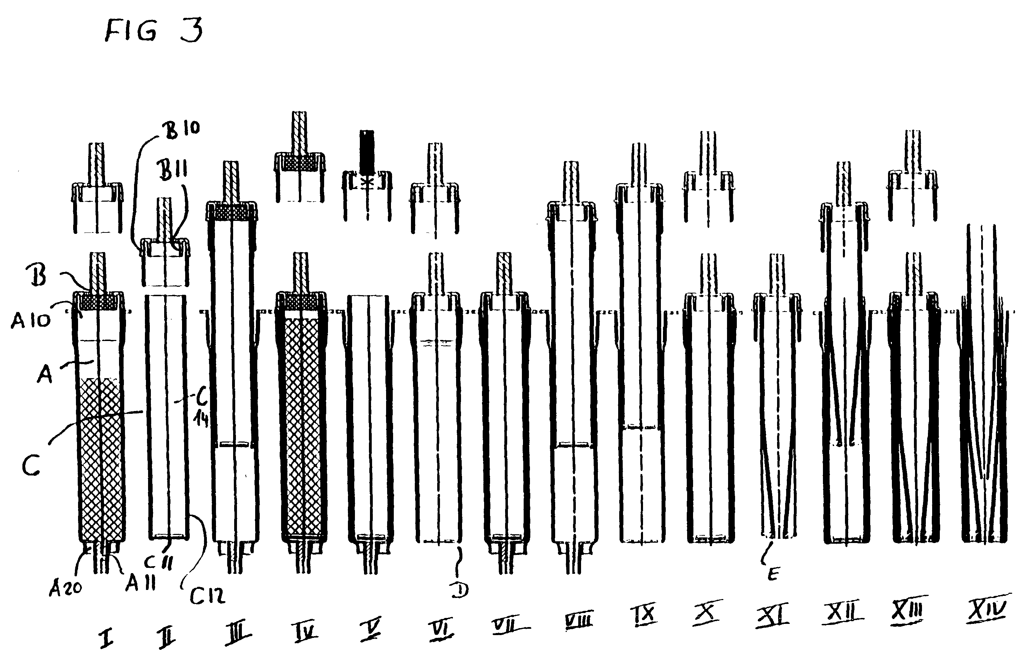

In FIG 3 ist ein erfindungsgemäßes Verfahren zur Isolierung von Nukleinsäuren gezeigt. Auf diese Figur wird bei der im folgenden Beispiel beschriebenen Schilderung eines Verfahrens Bezug genommen. Das Probengefäß befindet sich in einer Aufnahme in Einheit 10, wobei bevorzugt am Probengefäß ein Steg (A20) vorgesehen ist, der der Innenform der Aufnahme angepaßt ist (z. B. konische Außenform). Die im Längsschnitt gezeigten Gefäße können auf einfache Weise spritzgußtechnisch aus Polypropylen hergestellt werden.

Ein Hauptvorteil der Erfindung ist, daß das System in weitem Umfang auf die Verwendung unterschiedlicher Größen von Magnetpartikeln adaptiert werden kann. Es ist relativ flexibel und in unterschiedlichsten Verfahren einsetzbar.

Durch das folgende Beispiel wird der Gegenstand der Erfindung näher erläutert:

### Beispiel 1

Bei dem erfindungsgemäßen Verfahren handelt es sich um ein Verfahren, dessen Grundzüge dem Fachmann aus der Nukleinsäurediagnostik bekannt sind. Soweit experimentelle Details im Folgenden nicht ausgeführt sind, wird vollinhaltlich auf Molecular Cloning, Herausgeber J. Sambrook et al., CSH 1989 Bezug genommen.

In einer besonderen Ausführungsform des erfindungsgemäßen Verfahrens für die Aufarbeitung nukleinsäurehaltiger Probenlösungen, werden folgende Arbeitsschritte durchgeführt (siehe FIG 3). In einem ersten Schritt (I) wird eine zellhaltige Probenflüssigkeit in einem Probegefäß (A) mit einem Material inkubiert, an welches die Zellen gebunden werden, aus denen Nukleinsäuren gewonnen werden sollen. Hierzu kann dieses Material entweder spezifische Bindeeigenschaften für die Oberfläche der Zellen aufweisen, z. B. durch Immobilisierung von Antikörpern gegen Oberflächenantigene oder ein Absorbermaterial (A16, nicht gezeigt), es kann jedoch auch ein Material mit Filtereigenschaften (A15, nicht gezeigt) vorgesehen sein, durch welches die Zellen zurückgehalten werden, wenn die Flüssigkeit durch das Material durchtritt, z. B. aus dem Probengefäß entfernt wird. Bedingungen für die Immobilisierung von Zellen an Oberflächen sind dem Fachmann bekannt, z. B. aus Methods in Enzymology Vol. 171, Biomembranes / Part R Transport Theory: Cell and Model Membranes, Edited by Sidney Fleischer, Becca Fleischer, Department of Molecular Biology, Vanderbilt University, Nashville, Tennessee, Seiten 444 ff oder 581 ff..

Während der Inkubation ist das Probengefäß bevorzugt durch einen Deckel (B) verschlossen, um aktiven bzw. passiven Kontaminationsschutz zu gewährleisten.

In einem weiteren Schritt wird die Flüssigkeit aus dem Probengefäß entfernt, während Zellen, deren Nukleinsäuren isoliert werden sollen, in an das Material gebundenem Zustand im Probengefäß zurückbleiben. Da es sich bei dem zellbindenden Material um partikuläre Materialien handelt, kann ein Zurückhalten dadurch erreicht werden, daß das Material magnetisch ist (Hersteller: Dynal, Oslo, Norwegen) und der Magnet von außen an das Probengefäß herangeführt wird. Die Flüssigkeit kann durch die Auslaßöffnung (A11) unter Anlegen eines leichten Vakuums, abgesaugt werden. Hierzu ist an der Auslaßöffnung ein Ventil vorgesehen, welches sich durch Anlegen von Unterdruck öffnet.

Zur weitergehenden Entfernung eventuell störender Probenbestandteile von den Zellen werden ein oder mehrere Waschschritte vorgesehen. Hierzu wird in das Probengefäß eine Waschflüssigkeit eingefüllt, in der sich eventuelle Verunreinigungen lösen, die jedoch die Bindung der Zellen an die Oberfläche des zellbindenden Materials nicht wesentlich beeinträchtigen. Solche Waschlösungen sind dem Fachmann z. B. aus den Zellseparationsprotokollen bzw. aus entsprechenden Reinigungskitsprotokollen für Nukleinsäuren bekannt. Sie richten sich im wesentlichen nach der Art der Bindung der Zellen an das Material.

Nachdem gegebenenfalls die letzte Waschlösung aus dem Probengefäß (A) abgesaugt wurde, werden die gereinigten, angereicherten Zellen mit einer geeigneten Lyseflüssigkeit zur Freisetzung der Nukleinsäuren aus den Zellen in Kontakt gebracht. Die Reagenzien dieser Lyselösung richten sich weitgehend nach der Art der immobilisierten Zellen (Rolfs et al.: PCR, Clinical Diagnostics and Research, Springer Verlag, 1992, S. 84 ff). Sofern es sich bei den Zellen um Bakterien handelt, enthält die Lyselösung bevorzugt Proteinase K zum Abbau der Zellwand. Gewünschtenfalls wird die Lyse durch Erhitzen bzw. Abkühlen sowie Mischen der Reaktionsmischung durch Schütteln des Probengefäßes unterstützt. Am Ende dieses Aufschlusses liegen die zu isolierenden Nukleinsäuren frei in der Lösung vor.

Auch während der Lyse ist das Reaktionsgefäß bevorzugt durch einen Deckel verschlossen, um Kontaminationen aus der Umgebung zu verhindern. Nach Ende der Lyse wird der Deckel, bevorzugt mit Hilfe einer entsprechenden mechanischen Vorrichtung, entfernt. Danach wird in das Probengefäß, welches eine Mischung von Abbauprodukten der Zellen sowie die Nukleinsäuren enthält, ein Formkörper (C) eingeführt, dessen äußere Kontur (C12) auf die innere Kontur (A17) des Probengefäßes abgestimmt ist. Dieser Formkörper ist hohl und in Richtung auf das Probengefäß und die Reaktionsmischung hin durch einen Filter (C11) (poröse Matrix) verschlossen. Die Einführung des Formkörpers (C) erfolgt bevorzugt mit Hilfe eines Bauelementes (B11) des Deckels (B), der außerdem ein Bauelement (B10) enthält, welches zum Verschluß des Probengefäßes geeignet ist. In diesem Fall wird der Formkörper mit dem Deckel ergriffen (II) und gleichzeitig mit dem Verschluß des Probengefäßes in das Probengefäß eingeführt. Während dieses Vorgangs wird außerdem die Reaktionsmischung durch den Filter (C11) in den Hohlraum (C14) des Formkörpers eindringen (IV). Durch das Vorsehen des Filters können einerseits große Partikel an dem Eintritt in den Hohlraum gehindert werden und andererseits wird wegen der nukleinsäurebindenden Eigenschaften schon während des Durchtritts der Reaktionsmischung eine Bindung der Nukleinsäuren an den Filter erreicht. In diesem Fall wird ein glasfaserhaltiges Filtermaterial gewählt.

In einem nächsten Schritt wird die verbleibende Lysereaktionsmischung aus der durch A und C gebildeten Vorrichtung entfernt durch Absaugen durch die Auslaßöffnung (A11) im Probengefäß. Auch die in den Hohlkörper (C14) des Formkörpers eingedrungene Lösung wird somit entfernt, so daß der Filter möglichst keine Flüssigkeitsreste mehr enthält. Danach wird der bisher verwendete Deckel (B) entfernt, wobei der Formkörper (C) zunächst im Probengefäß verbleibt (eingerastet) (V).

Gleichzeitig oder anschließend wird ein Elutionsgefäß (D) zur Aufnahme des Formkörpers (C) vorbereitet (entweder im erfindungsgemäßen System oder außerhalb). Ein gegebenenfalls auf diesem Gefäß befindlicher Deckel wird entfernt (VI). Bevorzugt wird vor Überführung des Formkörpers (C) in das Elutionsgefäß (D) eine Elutionslösung in das Elutionsgefäß vorgelegt, z. B. einpipettiert. Die Zusammensetzung der Elutionslösung richtet sich nach der Art der Bindung der Nukleinsäuren an das Material im Filter (C). Sie enthält Reagenzien, unter deren Einwirkung die immobilisierten Nukleinsäuren von dem Material eluiert, d. h. gelöst, werden. Der ursprünglich das Elutionsgefäß verschließende Deckel (B) wird auf das Probengefäß (A) mit dem Formkörper (C) aufgesteckt (VII).

Zur Entnahme des Formkörpers (C) aus dem Probengefäß (A) wird der Formkörper (C) mit dem Deckel (B) entfernt (VIII). Die Kombination aus Deckel und Formkörper wird anschließend in das Elutionsgefäß eingeführt (IX). Bevorzugt enthält der Formkörper (C) Mittel (C13, nicht gezeigt) zur Fixierung des Formkörpers im Elutionsgefäß (D), die bewirken, daß der Formkörper nur unter Zerstörung des Formkörpers (C) oder des Gefäßes (D) oder mit einer Kraft, die größer ist als die Kraft die zur Lösung des Deckels (B) vom Formkörper (C) erforderlich ist, aus dem Gefäß (D) entfernt werden kann. Eine Entfernung des Formkörpers aus dem Elutionsgefäß ist nicht beabsichtigt.

Während des Eindringens des Formkörpers (C) in das Elutionsgefäß dringt die vorgelegte Elutionslösung in den Filter (C11) und die löst die immobilisierte Nukleinsäure von der festen Matrix ab. Je nach Menge der vorgelegten Elutionslösung wird entweder nur der Filter mit der Elutionslösung getränkt oder dringt die Elutionslösung mit den wieder gelösten Nukleinsäuren in den Hohlkörper (C14) ein. Damit die Elution der Nukleinsäuren möglichst vollständig verläuft, sollte die Innenkontur des Elutionsgefäßes möglichst dicht an die Außenkontur des Formkörpers angepaßt sein.

In einem folgenden Schritt wird der Deckel (B) von der Kombination aus Formkörper (C) und Elutionsgefäß (D) entfernt (X). Er wird benutzt, um einen Stempel (E) aufzunehmen (XI) und in den Hohlraum des Formkörpers (C) einzuführen (XII).Dieser Deckel greift von innen in den Stempel (E). Der Stempel wird so kräftig gegen den Filter (C11) gepreßt, daß Flüssigkeit aus dem Filter durch eine in der Andrucksfläche befindliche Öffnung in einen Innenraum des Stempels eindringt. Dieser Vorgang ist besonders effektiv, wenn die Andrucksfläche in ihrer äußeren Kontur zumindest in dem Bereich, in dem die Auspressung stattfinden soll, an die innere Kontur des Formkörpers (C) angepaßt ist. Der Stempel (E) kann bevorzugt in dieser Lage, z. B. durch Einrasten, fixiert werden. Da die so gebildete Vorrichtung durch den Deckel relativ gut verschlossen ist, kann die nukleinsäurehaltige Lösung in der Vorrichtung aufbewahrt werden.

Zur Entnahme einer gewünschten Menge an Nukleinsäurelösung kann der Deckel entfernt (XIII) und über eine Öffnung des Innenraums des Stempels die gewünschte Menge entnommen, z. B. in einem Pipettiervorgang (XIV). Anschließend kann der Deckel wieder aufgesetzt werden.

Im Folgenden wir das zu dem geschilderten Verfahren passende Ablaufschema angegeben.

| **Gerät** | **Anwender** |
|---|---|
| automatisch (programmgesteuert) | manuell |
| - temperieren | - pipettieren |
| - absaugen | - Tubes, Glasvlieseinsatz, Back-Up Gefäß auf Gerät plazieren |
| - separieren (magn. Festphase) | |
| - mischen/resuspendieren | |

Manuelle Arbeitsschritte sind fettgedruckt dargestellt. Nicht-manuelle Arbeitsschritte oder Teilabläufe werden durch Betätigen beispielsweise einer Taste aufgerufen.

Im Folgenden werden das Probengefäß A als Tube, Elutionsgefäß D als Back-Up-Gefäß, Formkörper C als Glasvlieseinsatz und Stempel E als Auspreßstempel bezeichnet.

| | | |
|---|---|---|
| Schritt # | Aktion | Zeit (s) |
| 1 | Tube 1* - 16 auf Reaktionsmodule plazieren | |
| 2 | pipettieren - Rezeptor (50-100µl) u. SA-beads (50-100 µl) in Tubes # 1 - 16 | |
| 3 | pipettieren - Probe (1.00 µl) in Tubes # 1 - 16 | |
| 4 | Deckel - Tubes verschiließen (16 Stück) | |
| | | |
| 5 | Mischen; Frequenz = 30 Hz (parallel) | 30 s |
| 6 | Inkubation; 9 = 4°C, nach Inkubation 9 = RT (parallel | 300-1.200 s |
| 6* | evtl. Mishen während Inkubation | |
| 7 | Magnet AKTIV (parallel) | 5 s |
| 8 | absaugen Waste (sequentiell 5 s) | 80 s |
| 9 | Magnet INAKTIV (parallel) | 5 s |
| | | |
| 10 | Deckel - Tube öffnen (16 Stück) | |
| 11 | 1. Waschschritt pipettieren - Waschlösung 500-1.000 µl (niedermolares Salz) in Tubes # 1 - 16 | |
| 12 | resuspendieren; Frequenz=30 Hz (parallel) | 5s |
| 13 | Magnet AKTIV (parallel) | 5 s |
| 14 | absaugen - Waste (sequentiell 5 s) | 80 s |
| 15 | Magnet INAKTIV (parallel) | 5 s |
| | | |
| 16 | 2. Waschschritt pipettieren - Waschlösung 500-1.000 µl (niedermolares Salz) in Tubes # 1 - 16 | |
| | | |
| 17 | resuspendieren; Frequenz = 30 Hz (parallel) | 5 s |
| 18 | Magnet AKTIV (parallel) | 5 s |
| 19 | absaugen - Waste (sequentiell 5 s) | 80 s |
| 20 | Magnet INAKTIV (parallel) | 5 s |
| | | |
| 20.1 | 3. Waschschritt (optional) pipettieren - Waschlösung 500-1.000 µl (niedermolares Salz) in Tubes # 1 -16 | |
| | | |
| 20.2 | resuspendieren; Frequenz = 30 Hz (parallel) | 5 s |
| 20.3 | Magnet AKTIV (parallel) | 5 s |
| 20.4 | absaugen - Waste (sequentiell 5 s) | 80 s |
| 20.5 | Magnet INAKTIV (parallel) | 5 s |
| | | |
| 21 | pipettieren - Lyse-Mix, Reagenz 1+2 (400 µl) Guanidinium Hydrochlorid oder Guanidinium Rhodanid und Proteinase K (25 µl) in Tubes # 1 -16 | |
| 22 | Deckel- Tubes verschließen (16 Stück) | |
| | | |
| 23 | resuspendieren; Frequenz = 30 Hz (parallel) | 5 s |
| 24 | Inkubation: 9=70°C | 600 s |
| | [Optional: Inkubation: 9 = 95°C bei pot. infekt. Proben] | 900 s |
| 25 | Inkubation: 9 = RT | 300 s |
| | | |
| 26 | Deckel -Tubes öffnen (16 Stück) | |
| 27 | pipettieren -Ethanol (Isopropanol) 200 µl in Tubes # 1 - 16 | |
| 28 | Deckel - Tubes verschließen (16 Stück) | |
| | | |
| 29 | Mischen; Frequenz = 30 Hz (parallel) | 30 s |
| | | |
| 30 | Deckel - Tubes öffnen (16 Stück) | |
| 31 | Glasvlies-Einsatz in Tube # 1 einsetzen in Tubes # 1-16 | |
| | | |
| 32 | absaugen - Waste (sequentiell 5 s) | 80 s |
| | | |
| 33 | pipettieren - Waschlösung 500 µl (chaotropes Salz/Ethanol) in Tubes # 1 - 16 | |
| | | |
| 34 | absaugen - Waste (sequentiell 5 s) | 80 s |
| | | |
| 35 | pipettieren - Waschlösung 500 µl (chaotropes Salz/Ethanol) in Tubes # 1 - 16 | |
| 36 | absaugen - Waste (sequentiell 5 s) | 80 s |
| | | |
| 37 | Back-Up-Gefäß auf Modul setzen (# 1- 16) | |
| 38 | pipettieren - Elutionsvolumen in Back-Up-Gefäß (100-200 µl) in Tubes # 1 - 16 | |
| 39 | Glasvlies-Einsatz von Tube # 1 in Back-Up-Gefäß überführen (# 1 -16) | |
| 40 | Auspreßstempel (# 1 - 16) in Back-Up-Gefäß hinein - Elution | |
| 41 | Verschließen Back-Up-Gefäß (16 Deckel) | |
| 42 | Tubes # 1 - 16 vom RM nehmen - Waste | |

Gewünschtenfalls werden die Absaugschläuche und die Ausnehmungen mittels einer Reinigungsflüssigkeit gespült und somit gereinigt (vor bzw. nach Durchführung des Verfahrens und in Abwesenheit der Probengefäße.

Eine weitere Ausführungsform eines Systems zur Freisetzung und Isolierung von Nukleinsäuren ist in den Figuren 4 bis 8 dargestellt.
- Figur 4:: Perspektivische Darstellung des Systems
- Figur 5:: Phantomzeichnung des Systems
- Figur 6:: Aufnahmeeinheit für Probenbearbeitungsgefäße
- Figur 7:: Querschnitt durch eine Aufnahmeposition für ein Probengefäß
- Figur 8:: Schieber mit Magneten

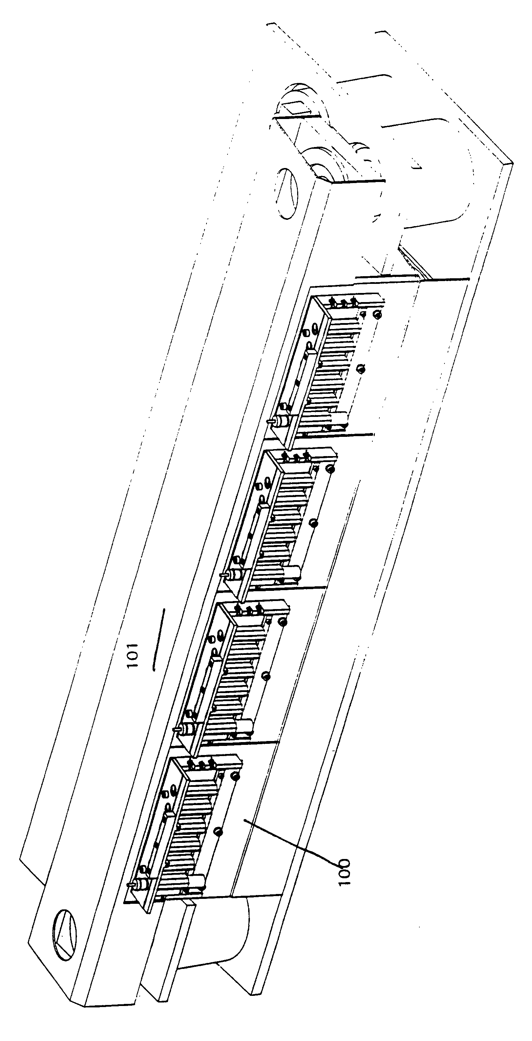

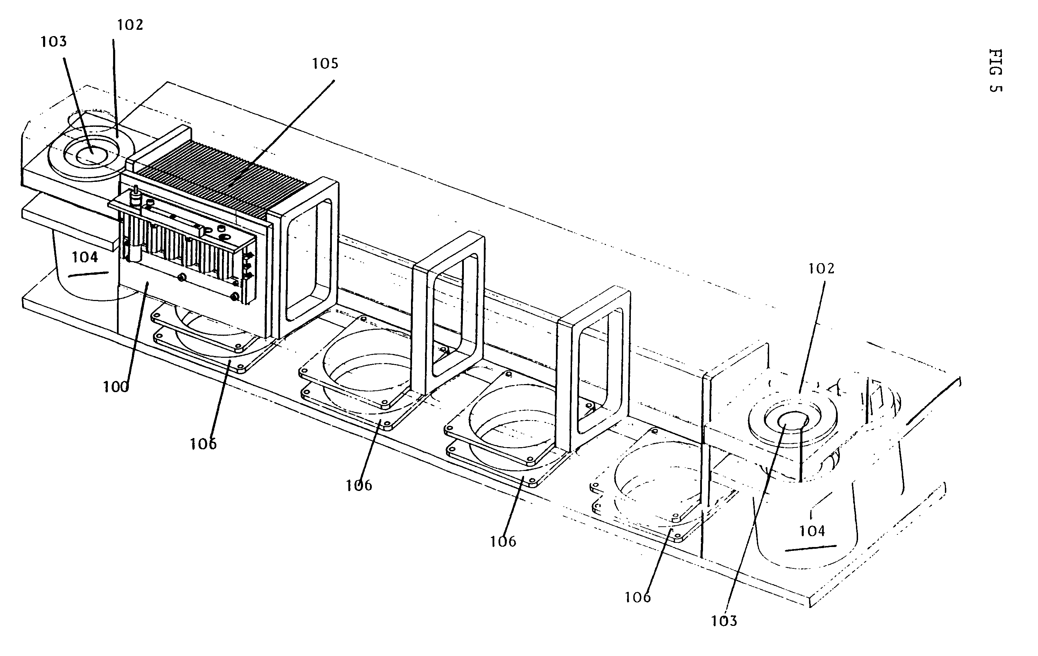

Das in Figur 4 dargestellte System besitzt 4 Aufnahmeeinheiten (100) für Probengefäße. Die Aufnahmeeinheiten (100) sind an einem Träger (101) befestigt. Durch eine Bewegung des Trägers (101) können die 4 Aufnahmeeinheiten (110) gemeinsam bewegt und geschüttelt werden. In der Phantomzeichnung (Figur 5) ist detaillierter zu erkennen, wie die Bewegung des Trägers (101) erfolgt. Der Träger besitzt an seinen 2 Enden jeweils eine kreisförmige Ausnehmung (102), in der ein Zapfen (103) läuft. Der Zapfen (103) ist exzentrisch auf der Achse eines Motors (104) angeordnet, so daß bei einer Drehung der Motorachse eine Verschiebung des Trägers in einer Ebene erfolgt. In der Phantomzeichnung (Figur 5) ist weiterhin zu erkennen, daß die Aufnahmeeinheit (100) Kühlrippen (105) besitzt. Durch Ventilatoren (106) im Boden des Systems wird zur Kühlung ein Luftstrom durch die Kühlrippen geblasen.

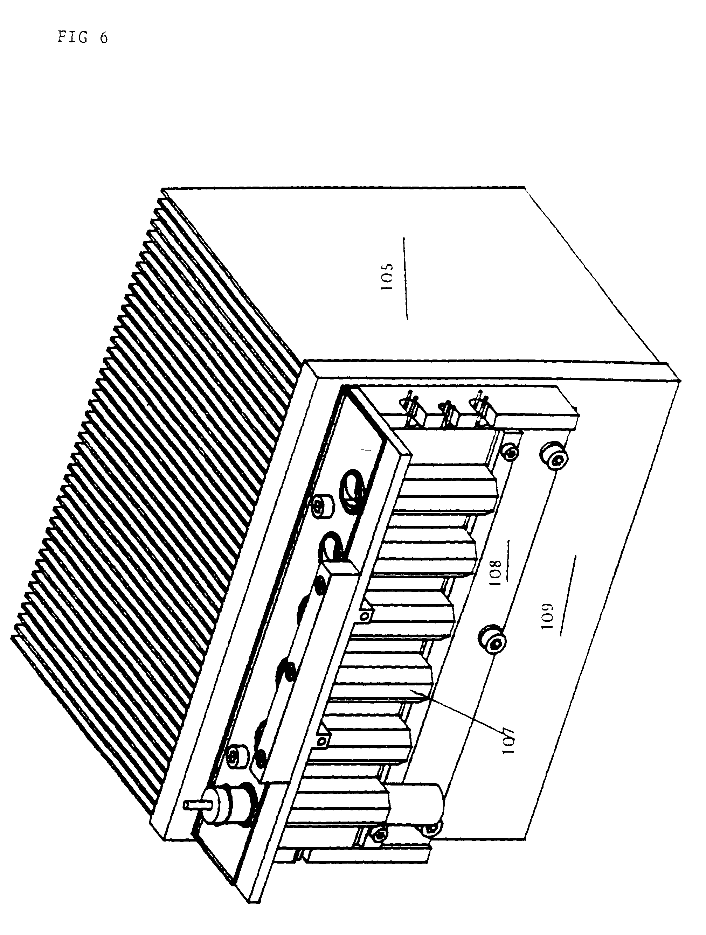

In Figur 6 ist eine Aufnahmeeinheit (100) detaillierter dargestellt. Die Aufnahmeeinheit besitzt 6 einzelne Aufnahmepositionen (107) zur Aufnahme von Probenbearbeitungsgefäßen. Die Aufnahmepositionen (107) sind über einen Rahmen (108) thermisch leitend mit dem Peltierelement (109) und dieses mit den Kühlrippen (105) verbunden. In dem Rahmen (108) befindet sich eine Widerstandsheizung zur Beheizung der Aufnahmepositionen sowie Temperaturdetektoren. Der Rahmen (108) ist auf einem Peltierelement (109) montiert, das in Wärmeaustausch mit den Kühlrippen (105) steht. Eine Erwärmung der Probenbearbeitungsgefäße erfolgt durch die im Rahmen untergebrachten Heizelemente. Zur Abkühlung der Probenbearbeitungsgefäße werden die Aufnahmepositionen (107) über das Peltierelement (109) gekühlt. Die vom Peltierelement auf der gegenüberliegenden Seite abgegebene Wärme wird über die Kühlrippen (105) abgeführt.

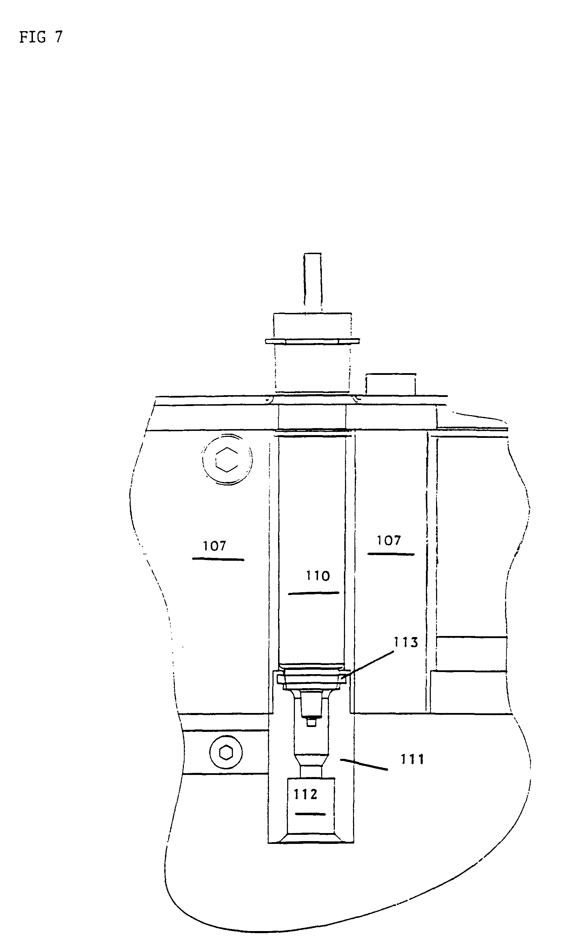

In Figur 7 ist ein Querschnitt durch eine einzelne Aufnahmeposition (107) dargestellt. Die Aufnahmeposition besitzt eine Bohrung, in der sich ein Probengefäß (110) befindet. Probengefäß und Bohrung sind so aufeinander abgestimmt, daß die Wandungen aneinander anliegen, um einen effizienten Wärmeübergang zu ermöglichen. Vorzugsweise besitzt das Probengefäß eine leichte Konizität, d. h. verjüngt sich zur Unterseite, da so bei Vorliegen einer entsprechenden Konizität der Aufnahmeposition ein guter Kontakt der Wandungen erreicht werden kann. Bevorzugt beträgt die Konizität 0,5 bis 1 °. Im unteren Bereich der Aufnahmeposition befindet sich ein Einsatz, der an seiner Unterseite eine Ausnehmung (112) besitzt, in welche die Olive eines Schlauches eingeschraubt werden kann. An seiner Oberseite besitzt der Einsatz (112) eine Ausnehmung, in die ein verjüngter Teil des Probengefäßes (110) eingeschoben wird. Um einen dichten Abschluß zwischen dieser Ausnehmung und der Verjüngung des Probengefäßes zu erzielen, befindet sich an dieser Stelle ein Dichtungsring (113) in Form eines O-Ringes, der die Verjüngung des Probengefäßes umfaßt. Zur Entnahme von Flüssigkeiten aus dem Probengefäß (110) wird mittels eines Schlauches, der an die Ausnehmung (112) angeschlossen ist, ein Unterdruck an das Probengefäß (110) angelegt.

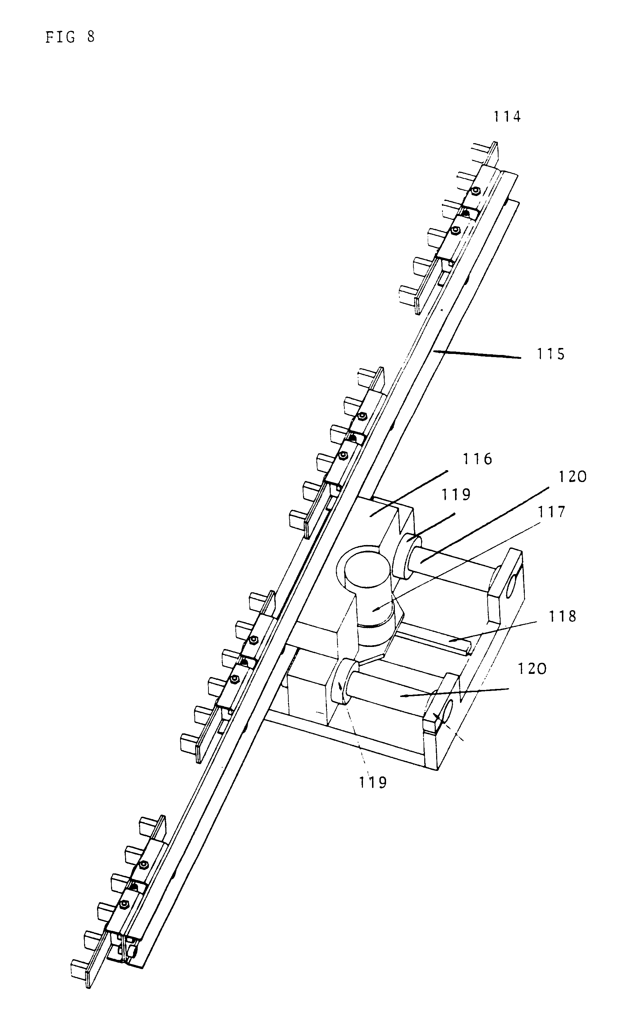

Die erfindungsgemäße Nutzung des Systems sieht es vor, daß an die Probengefäße (110), während sie sich in den Aufnahmepositionen (107) befinden, ein Magnetfeld angelegt werden kann, um magnetische Partikel festzuhalten. Zu diesem Zweck wird an die Aufnahmepositionen (107) die in Figur 8 dargestellte Anordnung von Magneten herangefahren.

Figur 8 zeigt eine bewegliche Anordnung von 4 x6 Magneten (114), entsprechend der Zahl von 4 Aufnahmeeinheiten (110) mit jeweils 6 Aufnahmepositionen (107). Die in Figur 8 dargestellte Anordnung besitzt eine Schiene (115), auf der 4 Einheiten von jeweils 6 Magneten montiert sind. Die Schiene (115) ist an dem Träger (116) befestigt, der weiterhin einen Motor (117) trägt. Auf der Achse des Motors ist ein Zahnrad (nicht dargestellt) montiert, das in die Zahnung (nicht dargestellt) einer Zahnstange (118) eingreift. Der Träger (116) ist durch Linearkugellager (119) auf Zylindern (120) verschiebbar angeordnet. In der Position, in der eine Abscheidung von Magnetpartikeln erfolgt, befinden sich die Magneten (114) mit ihren Stirnflächen direkt an den abgeflachten Seiten (121) der Aufnahmepositionen (107). Um die Magnete von den Aufnahmepositionen wegzubewegen, wird der Motor (117) aktiviert und der Träger samt der Schiene (115) linear verschoben.

### Bezugszeichenliste

- **A**: **Probengefäß**
10 Einlaßöffnung
11 Auslaßöffnung
17 innere Form
19 Außenform
20 umlaufender Steg
22 Element zur Fixierung von weiteren Funktionselementen
- **B**: **Deckel**
10 Bauelement zum Verschließen des Probengefäßes A
11 Bauelement zum Ergreifen des Formkörpers C
- **C**: **Formkörper**
11 poröse Matrix
12 äußere Kontur
13 Mittel zur Fixierung des Formkörpers im Elutionsgefäß
14 Hohlkörper
15 Mittel zur Befestigung eines Deckels
16 innere Kontur
17 Mittel zur Fixierung eines Stempels E, umlaufend
18 umlaufender Steg, abbrechbar
19 Rand
- **D**: **Elutionsgefäß**
12 Einrastkerbe
- **E**: **Stempel**
10 Andrucksfläche
11 Außenkontur
12 Innenraum
13 Öffnungen in der Andrucksfläche
14 Entnahmeöffnung
15 Dichtung
16 Einrastring
17 Aussparung

### Gerät

- 1: Rahmen
- 10: Aufnahmeeinheit für Probengefäße
- 11: Schwingungsdämpfer
- 12: Vertiefung/Kavität
- 13: Sockel
- 14: Inlet zum Heizen und Kühlen von A

- 20: Thermostatoreinheit zur Thermostatisierung von Probengefäßen
- 21: Kühl-/Heizmittel Leitung

- 30: Schütteler für Probengefäße

- 40: Abscheider zur magnetischen Abscheidung von Magnetpartikeln
- 41: Achsen zum Drehen der Magnetsegmente
- 42: Magnetsegmente

- 50: Vakuumpumpe/Pumpeneinheit
- 51: (Unterdruck)schlauch

- 100: Aufhahmeeinheit für Probengefäße
- 101: Träger
- 102: kreisförmige Ausnehmung
- 103: Zapfen
- 104: Motor
- 105: Kühlrippen
- 106: Ventilator
- 107: Aufnahmeposition
- 108: Rahmen
- 109: Peltierelement
- 110: Probengefäß
- 111: Einsatz
- 112: Ausnehmung
- 113: Dichtungsring
- 114: Magnet
- 115: Schiene
- 116: Träger
- 117: Motor
- 118: Zahnstange
- 119: Linearkugellager
- 120: Zylinder

## Patentansprüche

1. Verfahren zur Freisetzung und Isolierung oder zur Freisetzung und Nachweis von Nukleinsäuren aus biologischen Kompartimenten einer Probe, enthaltend die Schritte:
- Inkubation der Probe in einem Probenbearbeitungsgefäß zusammen mit Magnetpartikeln, welche die biologischen Kompartimente binden können, unter Schütteln des Probenbearbeitungsgefäßes,
- Positionierung eines Magneten in der Nähe des Gefäßes, so daß die Magnetpartikel an der Gefäßwand festgehalten werden,
- Entfernen der resultierenden Flüssigkeit aus dem Gefäß,
- Resuspension der Magnetpartikel in einer zweiten Flüssigkeit durch
a) Entfernen des Magneten aus der Nähe des Gefäßes, so daß die Magnetpartikel nicht mehr durch den Magneten an der Gefäßwand festgehalten werden und gleichzeitig
b) Schütteln des Gefäßes,
- Aufschluß der biologischen Kompartimente unter Herstellung einer Aufschlußmischung,
- Erwärmung der Aufschlußmischung,
- Abkühlung der Mischung unter Bedingungen, die die Isolierung oder Hybridisierung der zu isolierenden oder nachzuweisenden Nukleinsäure ermöglichen,
**dadurch gekennzeichnet, daß** das Festhalten der Magnetpartikel an der Gefäßwand vor dem Aufschluß der biologischen Kompartimente erfolgt und daß die Flüssigkeit durch eine untere Auslaßöffnung aus dem Gefäß entfernt wird.

2. Verfahren gemäß Anspruch 1, **dadurch gekennzeichnet, daß** während der genannten Schritte die Nukleinsäuren nicht aus dem Gefäß entfernt werden.

3. Verfahren gemäß Anspruch 1, **dadurch gekennzeichnet, daß** die genanriten Schritte in einem einzigen Reaktionsblock stattfinden.

4. Verfahren gemäß Anspruch 1, **dadurch gekennzeichnet, daß** die magnetischen Partikel eine Größe von mehr als 2,8 µm haben.

5. System zur Freisetzung und Isolierung von Nukleinsäuren aus biologischen Proben mit Magnetpartikeln enthaltend die Einheiten
a) eine Aufnahmeeinheit (10, 100) zur Aufnahme eines oder mehrerer Probenbearbeitungsgefäße (A),
b) eine Thermostateinheit (20) zur Thermostatisierung der Probenbearbeitungsgefäße (A) und der darin enthaltenen Flüssigkeiten,
c) einen Schüttler (30) zum Schütteln der Probenbearbeitungsgefäße (A),
d) einen Abscheider (40) zur magnetischen Abscheidung der Magnetpartikel an einer Wand jedes Probenbearbeitungsgefäßes (A),
wobei die unter a), b), c) und d) genannten Einheiten in aufeinander abgestimmter Kopplung in einem einzigen Reaktionsblock integriert sind und
die ein oder mehrere Probenbearbeitungsgefäße (A) eine untere Auslaßöffnung (A11) aufweisen, die bei Anordnung der Probenbearbeitungsgefäße in der Aufnahmeeinheit mit einer Saugvorrichtung verbunden sind.

6. System gemäß Anspruch 5, **dadurch gekennzeichnet, daß** die Saugvorrichtung eine Pumpeneinheit (50) zur Entfernung von Flüssigkeit aus dem Probenbearbeitungsgefäß (A) beinhaltet.

7. System gemäß Anspruch 5 oder 6, **dadurch gekennzeichnet, daß** der Abscheider (40) und die Aufnahmeeinheit (10) relativ zueinander beweglich gelagert sind.

8. System gemäß Anspruch 5, **dadurch gekennzeichnet, daß** die Aufnahmeeinheit (10, 100) durch einen Exzenterantrieb innerhalb einer Ebene bewegt wird, die im wesentlichen senkrecht zur Achse bzw. der Probenbearbeitungsgefäße ist.

9. System gemäß Anspruch 5, **dadurch gekennzeichnet, daß** die Thermostatoreinheit eine Widerstandsheizung sowie ein Peltierelement beinhaltet.

10. System gemäß Anspruch 5, bei dem Probenbearbeitungsgefäße und Aufnahmepositionen eine aufeinander abgestimmte Konizität aufweisen.

11. System gemäß Anspruch 5, **dadurch gekennzeichnet, daß** mittels der Saugvorrichtung der Inhalt des Probenbearbeitungsgefäßes in den Abfall überführt wird.

12. System gemäß Anspruch 5, **dadurch gekennzeichnet, daß** eine Dichtung das Ansaugen von Luft zwischen Probenbearbeitungsgefäß und Inlet (14) verhindert.

13. System gemäß Anspruch 6, **dadurch gekennzeichnet, daß** die Saugvorrichtung im wesentlichen aus einer Kolbenhubpumpe besteht.

14. System gemäß Anspruch 13, **dadurch gekennzeichnet, daß** das System mehrere Probenbearbeitungsgefäße besitzt, aus denen ein paralleles oder sequentielles Absaugen erfolgt.

15. System gemäß Anspruch 6, **dadurch gekennzeichnet, daß** die Saugvorrichtung im wesentlichen aus mindestens einer Schlauchpumpe besteht.

## Claims

1. Method for releasing and isolating or for isolating and detecting nucleic acids from biological compartments of a sample comprising the steps:
- incubating the sample in a sample processing vessel together with magnetic particles which can bind the biological compartments while the sample processing vessel is shaken,
- positioning a magnet near the vessel so that the magnetic particles are held against the vessel wall,
- removing the resulting liquid from the vessel,
- resuspending the magnetic particles in a second liquid by
a) removing the magnet from the vicinity of the vessel so that the magnetic particles are no longer held against the vessel wall by the magnet and simultaneously
b) shaking the vessel
- lysing the biological compartments to produce a lysis mixture,
- heating the lysis mixture,
- cooling the mixture under conditions that enable the isolation or hybridization of the nucleic acid to be isolated or detected,
**characterized in that** the magnetic particles are held against the vessel wall before lysing the biological compartments and that the liquid is removed from the vessel through a lower outlet opening.

2. Method as claimed in claim 1, **characterized in that** the nucleic acids are not removed from the vessel during the said steps.

3. Method as claimed in claim 1, **characterized in that** the said steps take place in a single reaction block.

4. Method as claimed in claim 1, **characterized in that** the magnetic particles have a size of more than 2.8 µm.

5. System for releasing and isolating nucleic acids from biological samples using magnetic particles comprising the components
a) a holding unit (10, 100) for holding one or more sample processing vessels (A),
b) a thermostat unit (20) to maintain the sample processing vessels (A) and their liquid contents at a constant temperature,
c) a shaker (30) for shaking the sample processing vessels (A),
d) a separation device (40) for magnetically depositing the magnetic particles on a wall of each sample processing vessel (A),
where the components mentioned under a), b), c) and d) are integrated in a matching connection into coupling in a single reaction block and the one or more sample processing vessels (A) have a lower outlet opening (A11) which are connected with a suction device when the sample processing vessels are positioned in the holding unit.

6. System as claimed in claim 5, **characterized in that** the suction device comprises a pump unit (50) for removing liquid from the sample processing vessel (A).

7. System as claimed in claim 5 or 6, **characterized in that** the separator (40) and the holding unit (10) are movably mounted relative to one another.

8. System as claimed in claim 5, **characterized in that** the holding unit (10, 100) is moved by means of an eccentric drive within a plane that is essentially perpendicular to the axis or to the sample processing vessels.

9. System as claimed in claim 5, **characterized in that** the thermostat unit comprises a resistance heating unit and a Pettier element.

10. System as claimed in claim 5, in which the sample processing vessels and holding positions have matching conicity.

11. System as claimed in claim 5, **characterized in that** the contents of the sample processing vessel are transferred to the waste by means of a suction device.

12. System as claimed in claim 5, **characterized in that** a seal prevents air from being sucked between the sample processing vessel and inlet (14).

13. System as claimed in claim 6, **characterized in that** the suction device essentially consists of a piston pump.

14. System as claimed in claim 13, **characterized in that** the system has a plurality of sample processing vessels out of which a simultaneous or sequential withdrawal by suction occurs.

15. System as claimed in claim 6, **characterized in that** the suction device is essentially composed of at least one peristaltic pump.

## Revendications

1. Procédé pour la libération et l'isolation ou pour la libération et la détection d'acides nucléiques dans des compartiments biologiques d'un échantillon, comprenant les étapes consistant à :
- incuber l'échantillon dans un récipient de traitement d'échantillons, en même temps que des particules magnétiques qui peuvent se lier aux compartiments biologiques, tout en secouant le récipient de traitement d'échantillons,
- placer un aimant à proximité du récipient de telle sorte que les particules magnétiques soient maintenues contre la paroi du récipient,
- retirer du récipient le liquide ainsi obtenu,
- remettre en suspension les particules magnétiques dans un deuxième liquide,
a) en éloignant l'aimant du récipient de telle sorte que les particules magnétiques ne soient plus retenues contre la paroi du récipient par l'aimant, et en même temps
b) en secouant le récipient,
- digérer les compartiments biologiques en réalisant un mélange de digestion,
- réchauffer le mélange de digestion,
- refroidir le mélange dans des conditions qui permettent l'isolation ou l'hybridation des acides nucléiques à isoler ou à détecter,
**caractérisé en ce que** le maintien des particules magnétiques contre la paroi du récipient s'effectue avant la dissolution des compartiments biologiques et **en ce que** le liquide est retiré du récipient par une ouverture de sortie inférieure.

2. Procédé selon la revendication 1, **caractérisé en ce que** les acides nucléiques ne sont pas retirés du récipient pendant lesdites étapes.

3. Procédé selon la revendication 1, **caractérisé en ce que** lesdites étapes ont lieu dans un seul bloc de réaction.

4. Procédé selon la revendication 1, **caractérisé en ce que** les particules magnétiques ont une taille supérieure à 2,8 µm.

5. Système pour la libération et l'isolation d'acides nucléiques dans des échantillons biologiques avec des particules magnétiques, comprenant les modules suivants :
a) un module de réception (10, 100) pour la réception d'un ou plusieurs récipients (A) de traitement d'échantillons,
b) un module thermostatique (20) pour thermostatiser les récipients (A) de traitement d'échantillons et les liquides qui y sont contenus,
c) un secoueur (30) pour secouer les récipients (A) de traitement d'échantillons,
d) un séparateur (40) pour la séparation magnétique des particules magnétiques contre une paroi de chaque récipient (A) de traitement d'échantillons,
les modules mentionnés en a), b), c) et d) étant intégrés dans un unique bloc de réaction en étant accouplés de manière harmonisée les uns par rapport aux autres et
le récipient ou la pluralité de récipients (A) de traitement d'échantillons présentent une ouverture inférieure de sortie (A11), qui est reliée à un dispositif d'aspiration lorsque les récipients de traitement d'échantillons sont disposés dans le module de réception.

6. Système selon la revendication 5, **caractérisé en ce que** le dispositif d'aspiration comprend un module de pompe (50) pour retirer le liquide du récipient (A) de traitement d'échantillons.

7. Système selon la revendication 5 ou 6, **caractérisé en ce que** le séparateur (40) et le module de réception (10) sont montés de manière à pouvoir se déplacer l'un par rapport à l'autre.

8. Système selon la revendication 5, **caractérisé en ce que** le module de réception (10, 100) est déplacé par un entraînement à excentrique dans un plan qui est essentiellement perpendiculaire à l'axe resp. des récipients de traitement d'échantillons.

9. Système selon la revendication 5, **caractérisé en ce que** le module thermostatique contient un chauffage à résistance ainsi qu'un élément Peltier.

10. Système selon la revendication 5, dans lequel les récipients de traitement d'échantillons et les logements de réception présentent des conicités adaptées l'une à l'autre.

11. Système selon la revendication 5, **caractérisé en ce que** le contenu du récipient de traitement d'échantillons est transféré dans les déchets par le dispositif d'aspiration.

12. Système selon la revendication 5, **caractérisé en ce qu'**un joint empêche l'aspiration de l'air entre le récipient de traitement d'échantillons et l'admission (14).

13. Système selon la revendication 6, **caractérisé en ce que** le dispositif d'aspiration se compose principalement d'une pompe aspirante-refoulante à piston.

14. Système selon la revendication 13, **caractérisé en ce que** le système possède plusieurs récipients de traitement d'échantillons qui sont aspirés en parallèle ou en séquences.

15. Système selon la revendication 6, **caractérisé en ce que** le dispositif d'aspiration se compose principalement d'au moins une pompe péristaltique.
